# EUROPEAN PATENT APPLICATION

(11) **EP 4 353 249 A1**
(43) Date of publication of application: **17.04.2024**
(21) Application number: 22810428.7
(22) Date of filing: 18.05.2022
(51) Int. Cl.: A61K 38/26, C07K 14/605, A61P 3/00

(54) **MULTI-AGONIST AND USE THEREOF**

(30) Priority: 26.05.2021 CN 202110576591
(71) Applicant: The United Bio-Technology (Hengqin) Co., Ltd., Zhuhai, Guangdong 519031 (CN)
(72) Inventor: HUANG, Liang, Zhuhai, Guangdong 519031 (CN); CAO, Chunlai, Zhuhai, Guangdong 519031 (CN); DENG, Huixing, Zhuhai, Guangdong 519031 (CN); ZHOU, Cui, Zhuhai, Guangdong 519031 (CN); HE, Xiuyi, Zhuhai, Guangdong 519031 (CN); LIU, Xiaoxiao, Zhuhai, Guangdong 519031 (CN); XIE, Xin, Zhuhai, Guangdong 519031 (CN)
(74) Representative: Michalski Hüttermann & Partner Patentanwälte mbB
(86) International application number: PCT/CN2022/093577
(87) International publication number: WO 2022/247701

(57) **Abstract**

The present application relates to the pharmaceutical and biological fields particularly relates to a polypeptide compound of general formula(I) with triple agonist activity on glucagon like peptide-1 receptor (GLP-1 R), glucose-dependent insulinotropic poypeptide receptor (GIP R), and glucagon receptor (GCG R), and use thereof in the treatment of metabolic syndrome.

## Description

### TECHNICAL FIELD

The present application relates to polypeptide compounds and use thereof in the pharmaceutical field. More specifically, the present application relates to polypeptide compound with triple agonist activity on glucagon like peptide-1 receptor (GLP-1 R), glucose-dependent insulinotropic polypeptide receptor (GIP R), and glucagon receptor (GCG R), and uses thereof in the treatment of metabolic syndrome.

### BACKGROUND TECHNOLOGY

Type II diabetes and obesity are increasingly becoming global diseases which affect human health. Obesity is the main cause of many chronic diseases such as diabetes, hypertension, heart disease, dyslipidemia, fatty liver disease, atherosclerosis, arthritis, stroke, neurodegenerative diseases, and diabetes can also lead to cardiovascular and cerebrovascular diseases and other complications. Currently, most of the hypoglycemic drugs on the market only have the effect of controlling blood sugar, but cannot improve the weight of an obese patient. Some drugs even have the side effect of increasing weight. Therefore, there is still an urgent need to develop drugs that can both reduce blood sugar and improve weight, and contain multiple beneficial effects to meet the needs of most obese and type II diabetes people.

Incretin is a type of polypeptide hormone secreted from the intestine after normal physiological eating stimulation. Early studies have found that it can stimulate the pancreatic islets β-Cells to secrete insulin as glucose levels rise after meals, regulate glucose homeostasis, and protect pancreatic islets β-Cells reduce weight by inhibiting appetite and delaying gastric emptying. GLP-1 and GIP are currently discovered as two types of pancreatic stimulating hormone.

GLP-1 is polypeptide with 31 amino acid and is expressed by the glucagon gene in intestinal mucosal L cells, and mainly acts on the GLP-1 receptor (GLP-1 R), stimulating insulin secretion, inhibiting glucagon secretion, protecting the pancreatic islets β-Cells, having a physiological function of regulating blood glucose homeostasis, while inhibiting food intake and gastric emptying through central nervous system signaling pathways, increasing satiety, and thus reducing weight. Exendin-4 is a GLP-1 analogue extracted from the salivary glands of African venomous lizards, which exhibits stronger GLP-1 receptor activation and similar GLP-1 effects. Compared to natural GLP-1, Exendin-4 has stronger resistance to DPP-4 and a longer plasma half-life in its body.

GIP is a single chain polypeptide with 42 amino acids which is produced by small intestinal mucosal K cells and mainly acts on GIP receptors (GIP R) in pancreatic islet cells and adipocytes. GIP glucose-dependent insulin secretion enhances pancreatic islets β-Cell quality, stimulates insulin secretion, inhibits gastric acid secretion, and slows down gastric peristalsis. In addition, it also stimulates the uptake and utilization of fatty acids by adipose tissue cells. GIP also has a physiological effect of promoting osteoblast differentiation, inhibiting osteoblast apoptosis, inhibiting bone resorption, increasing bone mineral density, and protecting bone.

GCG is a polypeptide containing 29 amino acids, is expressed and secretion by the glucagon proto-gene in the pancreatic islets α-cells, which mainly acts on the glucagon receptor (GCG R) distributed in the liver and kidneys, stimulating liver glycogen breakdown, raising blood sugar, activating lipase, promoting fat decomposition, while inhibiting liver fat synthesis and strengthening fatty acid oxidation. The research results indicate that GCG has a certain effect on reducing food intake, increasing energy consumption of adipose tissue, and reducing body fat. The appropriate effect of GCG that raise blood sugar can provide feedback on the regulation of insulin and reduce the occurrence of hypoglycemic events.

Aiming at the effect of incretin, GLP-1 receptor agonists such as Exenatide, Lisenatide, Liraglutide, Dulaglutide and Semaglutide have been successfully developed for the treatment of type II diabetes. In addition, liraglutide has been successfully developed for weight loss, and semaglutide is also undergoing clinical research on obesity indications. The advantage of GLP-1 analogues is that they can reduce blood glucose and also have cardiovascular benefits and weight management effects. However, at present, the weight loss of the single GLP-1 receptor agonist is still less than 10%, and there are obvious dose related gastrointestinal side effects (mainly nausea, vomiting, diarrhea). There is still urgently need for more effective therapeutic drugs of weight loss for the populations suffered from the metabolic diseases with complex conditions, such as obese type II diabetes, nonalcoholic fatty liver disease/nonalcoholic steatohepatitis (NAFLD/NASH), diabetes and obesity with cardiovascular risk.

According to the physiological effects of GLP-1, GIP and GCG, currently many studies have confirmed that activating both or three of their receptors simultaneously can achieve better therapeutic effects on diabetes and obesity than activating GLP-1R alone. It is reported that hyperglycemia, obesity, and insulin resistance hinder the effects of GIP R and it's signaling pathways. However, as blood sugar decreases and insulin resistance improve, the role of GIP in promoting insulin secretion and improving pancreatic islet function can be improved. In addition to promoting glycogen breakdown and raising blood sugar, GCG can also promote fat breakdown, inhibit liver fat synthesis, and exert the effect of lowering blood lipids and body weight. However, the synergistic inhibition of GLP-1 is required to suppress its blood sugar raising effect. Therefore, through the "vanguard" effect of GLP-1, reducing blood sugar and improving insulin resistance can further enhance the insulin secretion promoting function and insulin sensitization synergistic effect of GIP, improve pancreatic islet function, further enhance the lipid decomposition effect of GCG, improve lipid metabolism, and enhance the weight loss effect.

With the disclosure of clinical data on GLP-1/GIP and GLP-1/GCG dual agonists, the impact of the distribution of GLP-1/GIP or GLP-1/GCG dual agonist activity among different receptors on the clinical therapeutic efficacy of drugs is gradually becoming clear.

For example, the Phase II clinical study results of Lilly's Tirzepatide (LY3298176) showed that a 1mg dose of Tirzepatide had almost no weight loss effect, and its hypoglycemic effect was significantly lower than that of Dulaglutide 1.5mg. Only when the dosage of Tirzepatide is increased to 5mg, it can produce a hypoglycemic and weight loss effect better than 1.5mg of Dulaglutide. This is likely due to Tirzepatide's tendency towards stronger GIP receptor agonist activity, while GLP-1 receptor activity EC₅₀ is only about 15% of wild-type GLP-1 activity EC₅₀. In order to pursue maximum efficacy, the dosage of Tirzepatide has been significantly increased (up to a maximum dose of 15mg), which may increase the safety risk of the drug.

On the other hand, due to the strong hyperglycemic effect of GCG, sufficient GLP-1 activity is required in the GLP-1/GCG receptor double agonists to balance and suppress the hyperglycemic effect of GCG, thereby ensuring the hypoglycemic effect. The relative activity EC₅₀ of GLP-1 and GCG receptors in AstraZeneca's GLP-1/GCG receptor double agonist MEDI0382 (Cotadutide) was 29% and 12.8%, respectively. The results of a 26-week clinical trial showed a dose-dependent reduction in body weight, blood lipids, and levels of ALT and AST by 100-300µg of Cotadutide compared to liraglutide, and the effect of 300 µg-dose group was significantly better than that of 1.8mg liraglutide. However, there was no significant advantage in the reduction of HbA1c compared to liraglutide in each dose group. Further, as the dosage of Cotadutide increased, the lowering effect of glycated hemoglobin in the 300µg group was actually worse than that in the 200µg group. This is likely due to the low GLP-1 receptor activity of the Cotadutide molecule as the dose increases, which is insufficient to suppress the effect of GCG.

An ideal long-acting GLP-1/GIP/GCG triple receptor agonist molecule that occurs once a week, twice a week, or three times a week should have the highest possible GLP-1R/GIPR activity and relatively controllable GCGR activity to ensure maximum weight loss and hypoglycemic effects. CN104902919A and CN111040022A disclosed a series of GLP-1/GIP/GCG R triple agonist molecules based on the structural modification of venomous lizard exopeptide-4 (exendin-4); and CN109071624A discloses a series of cyclic peptide molecules combined with long-acting conjugates to form molecules. In addition, WO2015067716A1, WO2019125929A1, and WO2019125938A1 have also disclosed some polypeptides that link fatty acids to the side chains of amino acids at position 17. These polypeptides all demonstrate the triple activation effect of GLP-1/GIP/GCG R and have the long-term action potential of once a week. The molecules disclosed in the above patent applications cannot exhibit sufficiently high activity on GLP-1 R/GIP R/GCG R at same time.

There is still room for further development of the three agonist molecules.

### SUMMARY OF THE INVENTION

In view of the above technical conditions, the present invention provides a novel polypeptide molecule with GLP-1/GIP/GCG R triple activation activity, which can be used in the treatment of type II diabetes, obesity, dyslipidemia, nonalcoholic fatty liver disease/nonalcoholic steatohepatitis and other related metabolic diseases.

The present application provides a GLP-1/GIP/GCG R triple agonist polypeptide compound with general formula (I), or salts or solvates thereof:

Y-Aib-X3-GT-X6-TSDYSI-X13-LDK-X17-AQ-Aib-AFIE-X25-LLE-X29-X30-PSS-X34-X35-PP-X38- S-R¹ (I) ,

wherein,
X3 is Q or H;
X6 is F, αMeF, or αMeF (2F);
X13 is αMeL, F, αMeF, or L;
X17 is ψ ;
X25 is Y or F;
X29 is T, S, G, or Aib;
X30 is G, H, R, or Aib;
X34 is G or Aib;
X35 is A, Q, Aib, or H;
X38 is Ac3c or P;
R¹ is NH₂ or OH, or pharmaceutically acceptable salts and/or esters thereof;
where, ψ is Lys with side chains modified by the following general formula (II):

   Y-Z (II),

   wherein
   Y is (AEEAc or Glu)a-(AEEAc or Glu)b-(AEEAc or Glu)c, where a, b, and c are independently 0 or 1, and a, b, and c are not zero at the same time (As an exemplary illustration, AEEAc-AEEAc-yGlu), and a carboxyl end of Y is connected to ε-amino of Lys on a side chain; and
   Z is-CO-(CH₂)ₘ-R², m is an integer between 6 and 24, and R² is selected from-COOH.

In the present application, as one of the embodiments, the compound of general formula (I) includes at least two specific amino acids at the following sites:
X13 is For α MeF;
X25 is F;
X29 is T or S;
X30 is H, R, or Aib;
X35 is Q, Aib, or H;
X38isAc3c.

In the present application, as one of the embodiments, the general formula (II) is AEEAc-AEEAc-γGlu-CO(CH₂)₁₈COOH.

In the present application, as one of the embodiments, the compound is selected from:

| Compound | Sequence |
|---|---|
| 1 | Y-Aib-QGT-αMeF-TSDYSI-αMeL-LDKK(AEEAc-AEEAc-γGlu-CO(C H2)18COOH)AQ-Aib-AFIEYLLETGPSSGAPP-Ac3c-S-NH2 |
| 2 | Y-Aib-QGT-αMeF-TSDYSI-αMeL-LDKK(AEEAc-AEEAc-γGlu-CO(C H2)18COOH)AQ-Aib-AFIEYLLETHPSSGAPP-Ac3c-S-NH2 |
| 3 | Y-Aib-QGT-αMeF-TSDYSI-αMeL-LDKK(AEEAc-AEEAc-γGlu-CO(C H2)18COOH)AQ-Aib-AFIEYLLESHPSSGAPP-Ac3c-S-NH2 |
| 4 | Y-Aib-QGT-αMeF(2F)-TSDYSIFLDKK(AEEAc-AEEAc-γGlu-CO(CH2 )18COOH)AQ-Aib-AFIEYLLEGGPSSGAPP-Ac3c-S-NH2 |
| 5 | Y-Aib-QGT-αMeF(2F)-TSDYSIFLDKK(AEEAc-AEEAc-γGlu-CO(CH2 )18COOH)AQ-Aib-AFIEFLLEGGPSSGAPP-Ac3c-S-NH2 |
| 6 | Y-Aib-HGT-αMeF(2F)-TSDYSI-αMeL-LDKK(AEEAc-AEEAc-γGlu-C O(CH2)18COOH)AQ-Aib-AFIEYLLE –Aib-HPSSGQPPPS-NH2 |
| 7 | Y-Aib-QGT-αMeF-TSDYSI-αMeL-LDKK(AEEAc-AEEAc-γGlu-CO(C H2)18COOH)AQ-Aib-AFIEYLLETGPSSG-Aib-PPPS-NH2 |
| 8 | Y-Aib-QGT-αMeF-TSDYSI-αMeL-LDKK(AEEAc-AEEAc-γGlu-CO(C H2)18COOH)AQ-Aib-AFIEYLLETGPSSGQPPPS-NH2 |
| 9 | Y-Aib-QGT-αMeF(2F)-TSDYSIFLDKK(AEEAc-AEEAc-γGlu-CO(CH2 )18COOH)AQ-Aib-AFIEFLLETGPSSGAPP-Ac3c-S-NH2 |
| 10 | Y-Aib-QGT-αMeF(2F)-TSDYSIFLDKK(AEEAc-AEEAc-γGlu-CO(CH2 )18COOH)AQ-Aib-AFIEFLLESHPSSGAPP-Ac3c-S-NH2 |
| 11 | Y-Aib-QGT-αMeF(2F)-TSDYSIFLDKK(AEEAc-AEEAc-γGlu-CO(CH2 )18COOH)AQ-Aib-AFIEFLLETGPSSGAPP-Ac3c-S-NH2 |
| 12 | Y-Aib-QGT-αMeF-TSDYSIFLDKK(AEEAc-AEEAc-γGlu-CO(CH2)18C OOH)AQ-Aib-AFIEFLLETGPSSGAPPPS-NH2 |
| 13 | Y-Aib-QGT-αMeF-TSDYSIFLDKK(AEEAc-AEEAc-γGlu-CO(CH2)18C OOH)AQ-Aib-AFIEYLLETGPSSGAPPPS-NH2 |
| 14 | Y-Aib-QGT-αMeF-TSDYSI-αMeL-LDKK(AEEAc-AEEAc-γGlu-CO(C H2)18COOH)AQ-Aib-AFIEFLLETGPSSGAPPPS-NH2 |
| 15 | Y-Aib-QGT-αMeF-TSDYSIFLDKK(AEEAc-AEEAc-γGlu-CO(CH2)18C OOH)AQ-Aib-AFIEFLLESHPSSGAPPPS-NH2 |
| 16 | Y-Aib-QGT-αMeF-TSDYSIFLDKK(AEEAc-AEEAc-γGlu-CO(CH2)18C OOH)AQ-Aib-AFIEFLLE-Aib-HPSSGAPPPS-NH2 |
| 17 | Y-Aib-QGT-αMeF(2F)-TSDYSIFLDKK(AEEAc-AEEAc-γGlu-CO(CH2 )18COOH)AQ-Aib-AFIEYLLETGPSSGAPPPS-NH2 |
| 18 | Y-Aib-QGT-αMeF(2F)-TSDYSIFLDKK(AEEAc-AEEAc-γGlu-CO(CH2 )18COOH)AQ-Aib-AFIEYLLE-Aib-HPSSGAPPPS-NH2 |
| 19 | Y-Aib-QGT-αMeF(2F)-TSDYSI-αMeL-LDKK(AEEAc-AEEAc-γGlu-C O(CH₂)₁₈COOH)AQ-Aib-AFIEFLLETGPSSGAPPPS-NH₂ |
| 20 | Y-Aib-QGT-αMeF(2F)-TSDYSI-αMeL-LDKK(AEEAc-AEEAc-γGlu-C O(CH2)18COOH)AQ-Aib-AFIEFLLE-Aib-HPSSGAPPPS-NH2 |
| 21 | Y-Aib-QGT-αMeF(2F)-TSDYSIFLDKK(AEEAc-AEEAc-γGlu-CO(CH2 )18COOH)AQ-Aib-AFIEFLLEGGPSSGQPPPS-NH2 |
| 22 | Y-Aib-QGT-αMeF(2F)-TSDYSI-αMeL-LDKK(AEEAc-AEEAc-γGlu-C O(CH2)18COOH)AQ-Aib-AFIEFLLEGGPSSGQPPPS-NH2 |
| 23 | Y-Aib-QGT-αMeF(2F)-TSDYSI-αMeL-LDKK(AEEAc-AEEAc-γGlu-C O(CH2)18COOH)AQ-Aib-AFIEYLLETGPSSGQPPPS-NH2 |
| 24 | Y-Aib-QGT-αMeF(2F)-TSDYSI-αMeL-LDKK(AEEAc-AEEAc-γGlu-C O(CH₂)₁₈COOH)AQ-Aib-AFIEFLLETGPSS-Aib-HPPPS-NH₂ |
| 25 | Y-Aib-QGT-αMeF(2F)-TSDYSI-αMeL-LDKK(AEEAc-AEEAc-γGlu-C O(CH₂)₁₈COOH)AQ-Aib-AFIEFLLEGRPSS-Aib-HPPPS-NH₂ |
| 26 | Y-Aib-QGT-αMeF-TSDYSI-αMeL-LDKK(AEEAc-AEEAc-γGlu-CO(C H2)18COOH)AQ-Aib-AFIEFLLESHPSSGAPPPS-NH2 |
| 27 | Y-Aib-QGT-αMeF-TSDYSI-αMeF-LDKK(AEEAc-AEEAc-γGlu-CO(C H2)18COOH)AQ-Aib-AFIEYLLESHPSSGAPPPS-NH2 |
| 28 | Y-Aib-QGT-αMeF-TSDYSI-αMeF-LDKK(AEEAc-AEEAc-γGlu-CO(C H2)18COOH)AQ-Aib-AFIEFLLES-Aib-PSSGAPP-Ac3c-S-NH2 |
| 29 | Y-Aib-QGT-αMeF-TSDYSIFLDKK(AEEAc-AEEAc-γGlu-CO(CH2)18C OOH)AQ-Aib-AFIEYLLES-Aib-PSSGAPP-Ac3c-S-NH2 |
| 30 | Y-Aib-QGT-αMeF-TSDYSI-αMeL-LDKK(AEEAc-AEEAc-γGlu-CO(C H2)18COOH)AQ-Aib-AFIEFLLET-Aib-PSSGAPP-Ac3c-S-NH2 |
| 31 | Y-Aib-QGT-αMeF(2F)-TSDYSI-αMeL-LDKK(AEEAc-AEEAc-γGlu-C O(CH2)18COOH)AQ-Aib-AFIEFLLE-Aib-HPSSGAPP-Ac3c-S-NH2 |
| 32 | Y-Aib-QGT-αMeF(2F)-TSDYSIFLDKK(AEEAc-AEEAc-γGlu-CO(CH2 )18COOH)AQ-Aib-AFIEYLLE-Aib-HPSSGAPP-Ac3c-S-NH2 |
| 33 | Y-Aib-QGT-αMeF-TSDYSI-αMeL-LDKK(AEEAc-AEEAc-γGlu-CO(C H2)18COOH)AQ-Aib-AFIEFLLESHPSS-Aib-HPP-Ac3c-S-NH2 |
| 34 | Y-Aib-QGT-αMeF(2F)-TSDYSI-αMeL-LDKK(AEEAc-AEEAc-γGlu-C O(CH2)18COOH)AQ-Aib-AFIEYLLETGPSSGQPP-Ac3c-S-NH2 |
| 35 | Y-Aib-QGT-αMeF-TSDYSI-αMeL-LDKK(AEEAc-AEEAc-γGlu-CO(C H2)18COOH)AQ-Aib-AFIEYLLETGPSSGQPP-Ac3c-S-NH2 |
| 36 | Y-Aib-QGT-αMeF-TSDYSI-αMeL-LDKK(AEEAc-AEEAc-γGlu-CO(C H2)18COOH)AQ-Aib-AFIEFLLEGGPSS-Aib-HPPPS-NH2 |
| 37 | Y-Aib-QGT-αMeF-TSDYSI-αMeL-LDKK(AEEAc-AEEAc-γGlu-CO(C H2)18COOH)AQ-Aib-AFIEFLLEGRPSS-Aib-HPPPS-NH2 |
| 38 | Y-Aib-QGT-αMeF(2F)-TSDYSI-αMeL-LDKK(AEEAc-AEEAc-γGlu-C O(CH₂)₁₈COOH)AQ-Aib-AFIEFLLETGPSS-Aib-HPPPS-NH₂ |
| 39 | Y-Aib-QGT-αMeF(2F)-TSDYSI-αMeL-LDKK(AEEAc-AEEAc-γGlu-C O(CH2)18COOH)AQ-Aib-AFIEFLLETRPSS-Aib-HPPPS-NH2 |
| 40 | Y-Aib-QGT-αMeF(2F)-TSDYSI-αMeL-LDKK(AEEAc-AEEAc-γGlu-C O(CH₂)₁₈COOH)AQ-Aib-AFIEFLLESHPSS-Aib-HPP-Ac3c-S-NH₂ |
| 41 | Y-Aib-QGT-αMeF(2F)-TSDYSILLDKK(AEEAc-AEEAc-γGlu-CO(CH2 )18COOH)AQ-Aib-AFIEFLLESHPSS-Aib-HPP-Ac3c-S-NH2 |
| 42 | Y-Aib-QGT-αMeF-TSDYSILLDKK(AEEAc-AEEAc-γGlu-CO(CH2)18C OOH)AQ-Aib-AFIEFLLESHPSS-Aib-HPP-Ac3c-S-NH2 |
| 43 | Y-Aib-QGT-αMeF(2F)-TSDYSI-αMeL-LDKK(AEEAc-AEEAc-γGlu-C O(CH2)18COOH)AQ-Aib-AFIEYLLESHPSS-Aib-HPP-Ac3c-S-NH2 |

In the present invention, as one of the embodiments, the compound is further selected from:

| compound | sequence |
|---|---|
| 2 | Y-Aib-QGT-αMeF-TSDYSI-αMeL-LDKK(AEEAc-AEEAc-γGlu-CO(C H2)18COOH)AQ-Aib-AFIEYLLETHPSSGAPP-Ac3c-S-NH2 |
| 3 | Y-Aib-QGT-αMeF-TSDYSI-αMeL-LDKK(AEEAc-AEEAc-γGlu-CO(C H2)18COOH)AQ-Aib-AFIEYLLESHPSSGAPP-Ac3c-S-NH2 |
| 5 | Y-Aib-QGT-αMeF(2F)-TSDYSIFLDKK(AEEAc-AEEAc-γGlu-CO(CH2 )18COOH)AQ-Aib-AFIEFLLEGGPSSGAPP-Ac3c-S-NH2 |
| 8 | Y-Aib-QGT-αMeF-TSDYSI-αMeL-LDKK(AEEAc-AEEAc-γGlu-CO(C H2)18COOH)AQ-Aib-AFIEYLLETGPSSGQPPPS-NH2 |
| 18 | Y-Aib-QGT-αMeF(2F)-TSDYSIFLDKK(AEEAc-AEEAc-γGlu-CO(CH2 )18COOH)AQ-Aib-AFIEYLLE-Aib-HPSSGAPPPS-NH2 |
| 19 | Y-Aib-QGT-αMeF(2F)-TSDYSI-αMeL-LDKK(AEEAc-AEEAc-γGlu-C O(CH₂)₁₈COOH)AQ-Aib-AFIEFLLETGPSSGAPPPS-NH₂ |
| 20 | Y-Aib-QGT-αMeF(2F)-TSDYSI-αMeL-LDKK(AEEAc-AEEAc-γGlu-C O(CH2)18COOH)AQ-Aib-AFIEFLLE-Aib-HPSSGAPPPS-NH2 |
| 21 | Y-Aib-QGT-αMeF(2F)-TSDYSIFLDKK(AEEAc-AEEAc-γGlu-CO(CH2 )18COOH)AQ-Aib-AFIEFLLEGGPSSGQPPPS-NH2 |
| 22 | Y-Aib-QGT-αMeF(2F)-TSDYSI-αMeL-LDKK(AEEAc-AEEAc-γGlu-C O(CH2)18COOH)AQ-Aib-AFIEFLLEGGPSSGQPPPS-NH2 |
| 23 | Y-Aib-QGT-αMeF(2F)-TSDYSI-αMeL-LDKK(AEEAc-AEEAc-γGlu-C O(CH₂)₁₈COOH)AQ-Aib-AFIEYLLETGPSSGQPPPS-NH₂ |
| 24 | Y-Aib-QGT-αMeF(2F)-TSDYSI-αMeL-LDKK(AEEAc-AEEAc-γGlu-C O(CH₂)₁₈COOH)AQ-Aib-AFIEFLLETGPSS-Aib-HPPPS-NH₂ |
| 25 | Y-Aib-QGT-αMeF(2F)-TSDYSI-αMeL-LDKK(AEEAc-AEEAc-γGlu-C O(CH₂)₁₈COOH)AQ-Aib-AFIEFLLEGRPSS-Aib-HPPPS-NH₂ |
| 26 | Y-Aib-QGT-αMeF-TSDYSI-αMeL-LDKK(AEEAc-AEEAc-γGlu-CO(C H2)18COOH)AQ-Aib-AFIEFLLESHPSSGAPPPS-NH2 |
| 31 | Y-Aib-QGT-αMeF(2F)-TSDYSI-αMeL-LDKK(AEEAc-AEEAc-γGlu-C O(CH2)18COOH)AQ-Aib-AFIEFLLE-Aib-HPSSGAPP-Ac3c-S-NH2 |
| 32 | Y-Aib-QGT-αMeF(2F)-TSDYSIFLDKK(AEEAc-AEEAc-γGlu-CO(CH2 )18COOH)AQ-Aib-AFIEYLLE-Aib-HPSSGAPP-Ac3c-S-NH2 |
| 33 | Y-Aib-QGT-αMeF-TSDYSI-αMeL-LDKK(AEEAc-AEEAc-γGlu-CO(C H2)18COOH)AQ-Aib-AFIEFLLESHPSS-Aib-HPP-Ac3c-S-NH2 |
| 34 | Y-Aib-QGT-αMeF(2F)-TSDYSI-αMeL-LDKK(AEEAc-AEEAc-γGlu-C O(CH2)18COOH)AQ-Aib-AFIEYLLETGPSSGQPP-Ac3c-S-NH2 |
| 36 | Y-Aib-QGT-αMeF-TSDYSI-αMeL-LDKK(AEEAc-AEEAc-γGlu-CO(C H2)18COOH)AQ-Aib-AFIEFLLEGGPSS-Aib-HPPPS-NH2 |
| 37 | Y-Aib-QGT-αMeF-TSDYSI-αMeL-LDKK(AEEAc-AEEAc-γGlu-CO(C H2)18COOH)AQ-Aib-AFIEFLLEGRPSS-Aib-HPPPS-NH2 |
| 38 | Y-Aib-QGT-αMeF(2F)-TSDYSI-αMeL-LDKK(AEEAc-AEEAc-γGlu-C O(CH₂)₁₈COOH)AQ-Aib-AFIEFLLETGPSS-Aib-HPPPS-NH₂ |
| 39 | Y-Aib-QGT-αMeF(2F)-TSDYSI-αMeL-LDKK(AEEAc-AEEAc-γGlu-C O(CH2)18COOH)AQ-Aib-AFIEFLLETRPSS-Aib-HPPPS-NH2 |
| 40 | Y-Aib-QGT-αMeF(2F)-TSDYSI-αMeL-LDKK(AEEAc-AEEAc-γGlu-C O(CH₂)₁₈COOH)AQ-Aib-AFIEFLLESHPSS-Aib-HPP-Ac3c-S-NH₂ |
| 41 | Y-Aib-QGT-αMeF(2F)-TSDYSILLDKK(AEEAc-AEEAc-γGlu-CO(CH2 )18COOH)AQ-Aib-AFIEFLLESHPSS-Aib-HPP-Ac3c-S-NH2 |
| 42 | Y-Aib-QGT-αMeF-TSDYSILLDKK(AEEAc-AEEAc-γGlu-CO(CH2)18C OOH)AQ-Aib-AFIEFLLESHPSS-Aib-HPP-Ac3c-S-NH2 |
| 43 | Y-Aib-QGT-αMeF(2F)-TSDYSI-αMeL-LDKK(AEEAc-AEEAc-γGlu-C O(CH2)18COOH)AQ-Aib-AFIEYLLESHPSS-Aib-HPP-Ac3c-S-NH2 |

In the present application, as one of the embodiments, in terms of activation ability of GLP-1 receptor stable transfected cells, compared to natural GLP-1, the compound has a relative activity of at least 30%, preferably at least 60%, more preferably at least 80%, and further preferably at least 100% in terms of activation ability of GLP-1 receptor.

In the present application, as one of the embodiments, in terms of activation ability of GLP-1 receptor stable transfected cells, compared to natural GIP, the compound has a relative activity of at least 100% and more preferably at least 150% in terms of activation ability of GIP receptor.

In the present application, as one of the embodiments, in terms of activation ability of GCG receptor stable transfected cells, compared to natural GCG, the compound has a relative activity of at least 10%, more preferably at least 30% in terms of activation ability of GCG receptor.

In the present application, as one of the embodiments, in terms of activation on the representative cell RIN-m5F of pancreatic islet tissue, compared to natural GLP-1 (7-37), the compound has a relative activity of at least 60%, preferably at least 80%, and more preferably at least 100% in terms of activation on RIN-m5F cell.

In the present application, as one of the embodiments, in terms of activation on a representative cell 3T3-L1 of adipose tissue, compared to natural GIP, the compound has a relative activity of at least 60%, and preferably at least 100% in terms of activation on 3T3-L1 cell.

In the present application, as one of the embodiments, in terms of activation on human primary liver cells representative of liver tissue, compared to natural GCG, the compound has a relative activity of at least 60%, and preferably at least 100% in terms of activation on liver cells.

The present application further provides a pharmaceutical composition, including any of the compounds or salts or solvates thereof according to any of the above aspects in an effective amount, and pharmaceutically acceptable excipients, diluents, carriers, or excipients.

In one aspect, the pharmaceutical composition is injection, lyophilized powder, tablet, pill, pastille, soft capsule, hard capsule, granule, powder, solution, suspension or syrup; and alternatively, the drug composition is in the form of microcapsule, microspheres, nanoparticle, or liposome.

In one aspect, the pharmaceutical composition is used for oral administration, inhalation administration, or parenteral administration, and the parenteral administration is selected from intraperitoneal, intramuscular, arterial, intravenous, subcutaneous, or intradermal injection administration.

In one aspect, the pharmaceutical composition is administered at a frequency of at least once a day, once a week, once two weeks, or once a month.

In one aspect, the pharmaceutical composition can also be used in combination with at least one of the following active substances for treatment, including anti-diabetes activators (such as insulin and its analogues, biguanides, sulfonylureas, thiazolidinediones α-Glucosidase inhibitors, DPP-4 inhibitors, SGLT2 inhibitors, dual SGLT1/SGLT2 inhibitors, GLP-1 receptor agonists, amylin and its analogues, GIP receptor agonists, GCG receptor agonists or antagonists, GLP-1/GIP receptor agonists, GLP-1/GCG receptor agonists, GIP/GCG receptor agonists, FGF-21 and its analogues, cholecystokinin B (CCKB) and its analogues, PYY (3-36) and its analogues, leptin and its analogues, Calcitonin and its analogues, lipid-regulating active drugs, PPAR- α, β, δ Agonists or regulators, antiplatelet aggregation activators, PCSK9 inhibitors, lipase inhibitors, anti-liver fibrotic or cirrhotic activators, anti-inflammatory activators. As one of the embodiments, the anti-diabetes activator includes insulin and its analogues, biguanides, sulfonylureas, thiazolidinediones, α- Glucosidase inhibitors, DPP-4 inhibitors, SGLT2 inhibitors, dual SGLT1/SGLT2 inhibitors, GLP-1 receptor agonists, or amylin and its analogues.

An application of any one of the compounds or salts or solvates thereof or any one of the pharmaceutical composition according to the present application in the preparation of drugs for promoting insulin secretion and lowering blood sugar.

An application of any one of the compounds or salts or solvates thereof or any one of the pharmaceutical composition according to the present application in the preparation of drugs for inhibiting ingestion, delaying gastric emptying, increasing energy consumption, and reducing body weight.

An application of any one of the compounds or salts or solvates thereof or any one of the pharmaceutical composition according to the present application in the preparation of drugs for reducing pancreatic islets β-cell apoptosis, increasing pancreatic islets β-cell number, and improving the function of pancreatic islet cells.

Use of any one of the compounds or salts or solvates thereof or any one of the pharmaceutical composition according to the present application in the preparation of drugs for improving blood lipids, reducing liver fat accumulation, inhibiting the development of liver inflammation, and preventing and treating non-alcoholic fatty liver disease.

Use of any one of the compounds or salts or solvates thereof or any one of the pharmaceutical composition according to the present application in the preparation of drugs for promoting the growth of brain neurons, eliminating neurotoxic substances, inhibiting inflammation development, and exerting neuroprotective effects.

Use of any one of the compounds or salts or solvates thereof or any one of the pharmaceutical composition according to the present application in the preparation of drugs for the prevention and/or treatment of metabolic disorders and related complications, preferably for the treatment of diabetes, obesity or non-alcoholic fatty liver disease.

Use of any one of the compounds or salts or solvates thereof or any one of the pharmaceutical composition according to the present application in the preparation of drugs for treating dyslipidemia and related diseases, and neurodegenerative diseases, including Parkinson's disease and Alzheimer's disease.

Use of any one of the compounds or salts or solvates thereof or any one of the pharmaceutical composition according to the present application in the preparation of drugs for treating bone diseases related to endocrine disorders, metabolic disorders, kidney disease, or the like, and the bone disease comprise osteoporosis and osteoarthritis.

Any one of the compounds according to the present application can be synthesized by solid phase synthesis.

In one aspect, the present application provides compounds or their salts or solvent complexes with the following sequence:
Compound 1 (SEQ ID NO:1 
Y-Aib-QGT-αMeF-TSDYSI-αMeL-LDKK(AEEAc-AEEAc-γGlu-CO(CH2)18COOH)AQ-
Aib-AFIEYLLETGPSSGAPP-Ac3c-S-NH2
Compound 2 (SEQ ID NO:2) 
Y-Aib-QGT-αMeF-TSDYSI-αMeL-LDKK(AEEAc-AEEAc-γGlu-CO(CH₂)₁₈COOH)AQ-
Aib-AFIEYLLETHPSSGAPP-Ac3c-S-NH₂
Compound 3 (SEQ ID NO:3) 
Y-Aib-QGT-αMeF-TSDYSI-αMeL-LDKK(AEEAc-AEEAc-γGlu-CO(CH2)18COOH)AQ-
Aib-AFIEYLLESHPSSGAPP-Ac3c-S-NH2
Compound 4 (SEQ ID NO:4) 
Y-Aib-QGT-αMeF(2F)-TSDYSIFLDKK(AEEAc-AEEAc-γGlu-CO(CH2)18COOH)AQ-Ai
b-AFIEYLLEGGPSSGAPP-Ac3c-S-NH2
Compound 5 (SEQ ID NO:5) 
Y-Aib-QGT-αMeF(2F)-TSDYSIFLDKK(AEEAc-AEEAc-γGlu-CO(CH2)18COOH)
AQ-Aib-AFIEFLLEGGPSSGAPP-Ac3c-S-NH2
Compound 6 (SEQ ID NO:6) 
Y-Aib-HGT-αMeF(2F)-TSDYSI-αMeL-LDKK(AEEAc-AEEAc-γGlu-CO(CH2)18COOH)
AQ-Aib-AFIEYLLE-Aib-HPSSGQPPPS-NH2
Compound 7 (SEQ ID NO:7) 
Y-Aib-QGT-αMeF-TSDYSI-αMeL-LDKK(AEEAc-AEEAc-γGlu-CO(CH2)18COOH)AQ-
Aib-AFIEYLLETGPSSG-Aib-PPPS-NH2
Compound 8 (SEQ ID NO:8) 
Y-Aib-QGT-αMeF-TSDYSI-αMeL-LDKK(AEEAc-AEEAc-γGlu-CO(CH₂)₁₈COOH)AQ-
Aib-AFIEYLLETGPSSGQPPPS-NH₂
Compound 9 (SEQ ID NO:9) 
Y-Aib-QGT-αMeF(2F)-TSDYSIFLDKK(AEEAc-AEEAc-γGlu-CO(CH2)18COOH)AQ-Aib-AF
IEFLLETGPSSGAPP-Ac3c-S-NH2
Compound 10 (SEQ ID NO:10) 
Y-Aib-QGT-αMeF(2F)-TSDYSIFLDKK(AEEAc-AEEAc-γGlu-CO(CH2)18COOH)AQ-Ai
b-AFIEFLLESHPSSGAPP-Ac3c-S-NH2
Compound 11 (SEQ ID NO:11) 
Y-Aib-QGT-αMeF-TSDYSIFLDKK(AEEAc-AEEAc-γGlu-CO(CH2)18COOH)AQ-Aib-AF
IEFLLETGPSSGAPP-Ac3c-S-NH2
Compound 12 (SEQ ID NO:12) 
Y-Aib-QGT-αMeF-TSDYSIFLDKK(AEEAc-AEEAc-γGlu-CO(CH2)18COOH)AQ-Aib-AF
IEFLLETGPSSGAPPPS-NH2
Compound 13 (SEQ ID NO:13) 
Y-Aib-QGT-αMeF-TSDYSIFLDKK(AEEAc-AEEAc-γGlu-CO(CH2)18COOH)AQ-Aib-AF
IEYLLETGPSSGAPPPS-NH2
Compound 14 (SEQ ID NO:14) 
Y-Aib-QGT-αMeF-TSDYSI-αMeL-LDKK(AEEAc-AEEAc-γGlu-CO(CH₂)₁₈COOH)AQ-
Aib-AFIEFLLETGPSSGAPPPS-NH₂
Compound 15 (SEQ ID NO:15) 
Y-Aib-QGT-αMeF-TSDYSIFLDKK(AEEAc-AEEAc-γGlu-CO(CH2)18COOH)AQ-Aib-AF
IEFLLESHPSSGAPPPS-NH2
Compound 16 (SEQ ID NO:16) 
Y-Aib-QGT-αMeF-TSDYSIFLDKK(AEEAc-AEEAc-γGlu-CO(CH2)18COOH)AQ-Aib-AF
IEFLLE-Aib-HPSSGAPPPS-NH2
Compound 17 (SEQ ID NO:17) 
Y-Aib-QGT-αMeF(2F)-TSDYSIFLDKK(AEEAc-AEEAc-γGlu-CO(CH2)18COOH)AQ-Ai
b-AFIEYLLETGPSSGAPPPS-NH2
Compound 18 (SEQ ID NO:18) 
Y-Aib-QGT-αMeF(2F)-TSDYSIFLDKK(AEEAc-AEEAc-γGlu-CO(CH2)18COOH)AQ-Ai
b-AFIEYLLE-Aib-HPSSGAPPPS-NH2
Compound 19 (SEQ ID NO:19) 
Y-Aib-QGT-αMeF(2F)-TSDYSI-αMeL-LDKK(AEEAc-AEEAc-γGlu-CO(CH₂)₁₈COOH)
AQ-Aib-AFIEFLLETGPSSGAPPPS-NH₂
Compound 20 (SEQ ID NO:20) 
Y-Aib-QGT-αMeF(2F)-TSDYSI-αMeL-LDKK(AEEAc-AEEAc-γGlu-CO(CH₂)₁₈COOH)
AQ-Aib-AFIEFLLE-Aib-HPSSGAPPPS-NH₂
Compound 21 (SEQ ID NO:21) 
Y-Aib-QGT-αMeF(2F)-TSDYSIFLDKK(AEEAc-AEEAc-γGlu-CO(CH2)18COOH)AQ-Ai
b-AFIEFLLEGGPSSGQPPPS-NH2
Compound 22 (SEQ ID NO:22) 
Y-Aib-QGT-αMeF(2F)-TSDYSI-αMeL-LDKK(AEEAc-AEEAc-γGlu-CO(CH₂)₁₈COOH)
AQ-Aib-AFIEFLLEGGPSSGQPPPS-NH₂
Compound 23 (SEQ ID NO:23) 
Y-Aib-QGT-αMeF(2F)-TSDYSI-αMeL-LDKK(AEEAc-AEEAc-γGlu-CO(CH2)18COOH)
AQ-Aib-AFIEYLLETGPSSGQPPPS-NH2
Compound 24 (SEQ ID NO:24) 
Y-Aib-QGT-αMeF(2F)-TSDYSI-αMeL-LDKK(AEEAc-AEEAc-γGlu-CO(CH2)18COOH)
AQ-Aib-AFIEFLLETGPSS-Aib-HPPPS-NH2
Compound 25 (SEQ ID NO:25) 
Y-Aib-QGT-αMeF(2F)-TSDYSI-αMeL-LDKK(AEEAc-AEEAc-γGlu-CO(CH₂)₁₈COOH)
AQ-Aib-AFIEFLLEGRPSS-Aib-HPPPS-NH₂
Compound 26 (SEQ ID NO:26) 
Y-Aib-QGT-αMeF-TSDYSI-αMeL-LDKK(AEEAc-AEEAc-γGlu-CO(CH₂)₁₈COOH)AQ-
Aib-AFIEFLLESHPSSGAPPPS-NH₂
Compound 27 (SEQ ID NO:27) 
Y-Aib-QGT-αMeF-TSDYSI-αMeF-LDKK(AEEAc-AEEAc-γGlu-CO(CH2)18COOH)AQ-
Aib-AFIEYLLESHPSSGAPPPS-NH2
Compound 28 (SEQ ID NO:28) 
Y-Aib-QGT-αMeF-TSDYSI-αMeL-LDKK(AEEAc-AEEAc-γGlu-CO(CH₂)₁₈COOH)AQ-
Aib-AFIEFLLES-Aib-PSSGAPP-Ac3c-S-NH₂
Compound 29 (SEQ ID NO:29) 
Y-Aib-QGT-αMeF-TSDYSIFLDKK(AEEAc-AEEAc-γGlu-CO(CH2)18COOH)AQ-Aib-AF
IEYLLES-Aib-PSSGAPP-Ac3c-S-NH2
Compound 30 (SEQ ID NO:30) Y-Aib-QGT-αMeF-TSDYSI-αMeL-LDKK(AEEAc-AEEAc-γGlu-CO(CH2)18COOH)AQ- Aib-AFIEFLLET-Aib-PSSGAPP-Ac3c-S-NH2
Compound 31 (SEQ ID NO:31) 
Y-Aib-QGT-αMeF(2F)-TSDYSI-αMeL-LDKK(AEEAc-AEEAc-γGlu-CO(CH2)18COOH)
AQ-Aib-AFIEFLLE-Aib-HPSSGAPP-Ac3c-S-NH2
Compound 32 (SEQ ID NO:32) 
Y-Aib-QGT-αMeF(2F)-TSDYSIFLDKK(AEEAc-AEEAc-γGlu-CO(CH2)18COOH)AQ-Ai
b-AFIEYLLE-Aib-HPSSGAPP-Ac3c-S-NH2
Compound 33 (SEQ ID NO:33) 
Y-Aib-QGT-αMeF-TSDYSI-αMeL-LDKK(AEEAc-AEEAc-γGlu-CO(CH2)18COOH)AQ-Aib-A
FIEFLLESHPSS-Aib-HPP-Ac3c-S-NH2
Compound 34 (SEQ ID NO:34) 
Y-Aib-QGT-αMeF(2F)-TSDYSI-αMeL-LDKK(AEEAc-AEEAc-γGlu-CO(CH2)18COOH)
AQ-Aib-AFIEYLLETGPSSGQPP-Ac3c-S-NH2
Compound 35 (SEQ ID NO:35) 
Y-Aib-QGT-αMeF-TSDYSI-αMeL-LDKK(AEEAc-AEEAc-γGlu-CO(CH₂)₁₈COOH)AQ-
Aib-AFIEYLLETGPSSGQPP-Ac3c-S-NH₂
Compound 36 (SEQ ID NO:36) 
Y-Aib-QGT-αMeF-TSDYSI-αMeL-LDKK(AEEAc-AEEAc-γGlu-CO(CH₂)₁₈COOH)AQ-
Aib-AFIEFLLEGGPSS-Aib-HPPPS-NH₂
Compound 37 (SEQ ID NO:37) 
Y-Aib-QGT-αMeF-TSDYSI-αMeL-LDKK(AEEAc-AEEAc-γGlu-CO(CH2)18COOH)AQ-
Aib-AFIEFLLEGRPSS-Aib-HPPPS-NH2
Compound 38 (SEQ ID NO:38) 
Y-Aib-QGT-αMeF(2F)-TSDYSI-αMeL-LDKK(AEEAc-AEEAc-γGlu-CO(CH2)18COOH)
AQ-Aib-AFIEFLLETGPSS-Aib-HPPPS-NH2
Compound 39 (SEQ ID NO:39) 
Y-Aib-QGT-αMeF(2F)-TSDYSI-αMeL-LDKK(AEEAc-AEEAc-γGlu-CO(CH2)18COOH)
AQ-Aib-AFIEFLLETRPSS-Aib-HPPPS-NH2
Compound 40 (SEQ ID NO:40) 
Y-Aib-QGT-αMeF(2F)-TSDYSI-αMeL-LDKK(AEEAc-AEEAc-γGlu-CO(CH2)18COOH)
AQ-Aib-AFIEFLLESHPSS-Aib-HPP-Ac3c-S-NH2
Compound 41 (SEQ ID NO:41) 
Y-Aib-QGT-αMeF(2F)-TSDYSILLDKK(AEEAc-AEEAc-γGlu-CO(CH2)18COOH)AQ-Ai
b-AFIEFLLESHPSS-Aib-HPP-Ac3c-S-NH2
Compound 42 (SEQ ID NO:42) 
Y-Aib-QGT-αMeF-TSDYSILLDKK(AEEAc-AEEAc-γGlu-CO(CH2)18COOH)AQ-Aib-A
FIEFLLESHPSS-Aib-HPP-Ac3c-S-NH2
Compound 43 (SEQ ID NO:43)

Y-Aib-QGT-αMeF(2F)-TSDYSI-αMeL-LDKK(AEEAc-AEEAc-γGlu-CO(CH2)18COOH)AQ-A
ib-AFIEYLLESHPSS-Aib-HPP-Ac3c-S-NH2,

GLP-1 R, GIP R, and GCG R have been found to be distributed and expressed in multiple metabolic related tissues, organs, and cells in the human body. For example, GLP-1 R, GIP R, and GCG R are simultaneously expressed in pancreatic islet tissue cells, with GLP-1 R being the most abundant receptor. For example, GLP-1 R and GIP R are found to be expressed in adipocytes, with the highest GIP R abundance. There have also been reports of the expression of GLP-1 R and GCG R in liver cells, with the highest abundance of GCG R. At the same time, the inventors were surprised to find that, although some compounds can have better EC₅₀ than wild-type polypeptides in single receptor stable transfected cells, even higher doses are required to fully stimulate tissue cells compared to wild-type polypeptides in the aforementioned tissue cells. This is likely related to the distribution tendency of multi agonist compounds across multiple receptors. Therefore, in order to achieve the maximum therapeutic effect, it is required that the three agonist molecules should be able to fully activate multiple receptors in tissue cells, and at least be able to effectively activate high abundance receptors in tissue cells. Therefore, the EC₅₀ of the three agonist molecules on various tissue cells should be at least better than the corresponding wild-type polypeptide GLP-1/GIP/GCG.

The compound of the present application has strong relative activity EC₅₀ against the three components in GLP-1/GIP/GCG R, and can fully stimulate the corresponding target organ tissue cells.

Preferably, the compound of the present application can promote insulin secretion and lower blood sugar. Preferably, it can also inhibit feeding, delay gastric emptying, increase energy consumption, and ultimately observe weight loss effects.

Preferably, the compound according to the present application can reduce pancreatic islets β-cell apoptosis, increase of number of pancreatic islets β-cells and improve the function of pancreatic islet cells.

Preferably, the compound according to the present application can also improve blood lipids, reduce liver fat accumulation, inhibit the development of liver inflammation, and prevent and treat non-alcoholic fatty liver disease.

Preferably, the compound according to the present application is expected to promote the growth of brain neurons, eliminate neurotoxic substances, inhibit the development of inflammation, and play a neuroprotective role.

Preferably, the compounds or compositions according to the present application can be used to prevent and/or treat metabolic disorders and related complications. It is preferably used to treat diabetes, obesity and nonalcoholic fatty liver disease.

Preferably, the compounds or compositions according to the present application can be used to treat dyslipidemia and related diseases, as well as neurodegenerative diseases such as Parkinson's disease and Alzheimer's disease.

Preferably, the compounds or compositions according to the present application can be used to treat bone diseases related to endocrine disorders, metabolic disorders, kidney disease, and other causes, such as osteoporosis and osteoarthritis.

The compound of the present application has significant stimulating effects on GLP-1, GIP, and GCG receptors.

The polypeptide compounds according to the present application can be synthesized and modified by those skilled in the art through known technical methods. For example, the polypeptide sequence skeleton of the polypeptide compound according to the present application can be prepared by methods such as synthesis.

The peptide skeleton of the compound according to the present application is chemically modified by fatty acid side chain groups on at least one site. Preferably, the compound can have stable peptides α-helical structure, and enhance albumin binding force, achieving improved stability of peptide compounds and extended peptide action time.

The compound according to the present application has agonistic activity on GLP-1 receptors, GIP receptors, and GCG receptors. The "agonistic activity" mentioned refers to the compound's ability to stimulate specific receptor cells to produce cAMP, which can be constructed by skilled individuals in the art to overexpress GLP-1 receptors, GIP receptors, or GCG receptors in host cells, pancreatic tissue cells, adipocytes, liver cells, etc. The receptor activation activity can be measured by the EC₅₀ value of cAMP produced by the compound stimulating receptor cells. The EC₅₀ value refers to the drug concentration value required to reach one half of the maximum activity (50% activity) of a compound in a specific measurement system.

In specific embodiments, the agonistic activity of the compound can be evaluated by evaluating the relative activity of specific natural compounds. The relative activity is the percentage of the ratio of the EC₅₀ value of a specific natural compound to the EC₅₀ value of the test compound.

Compared to existing compounds, the GLP-1/GIP/GCG R triple agonist polypeptide molecule provided by the present application has better GLP-1 R, GIP R, GCG R, and tissue cell relative activity EC₅₀.

### Definition

The 'relative activity EC₅₀' refers to the ratio of the EC₅₀ values of the corresponding wild-type positive peptides human GLP-1 (7-37), human GIP, and human GCG to the EC₅₀ values of the compounds of the present application.

The amino acids in the compound sequence of the present application originate from natural amino acids or related amino acid variants and/or derivatives. The abbreviations and codes of the natural amino acids mentioned are based on general rules familiar to those skilled in the art. Aib, α-MeF, α-MeF (2F), α-The chemical structural formula of MeL and Ac3c is as follows:

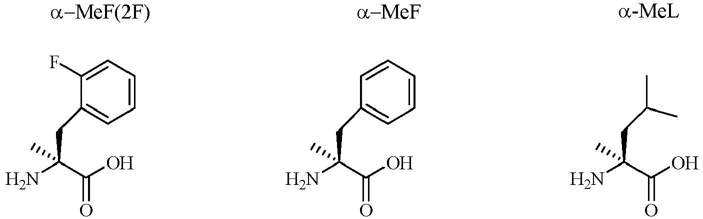

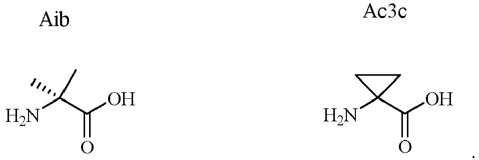

Unless otherwise defined, all technical and scientific terms used herein have the same meanings as those commonly understood by those skilled in the art. In case of conflict, this document, including the definition, shall prevail. The preferred methods and materials are described below, but methods and materials similar or equivalent to those described herein can be used to implement or test the present application. The materials, methods, and examples disclosed herein are only illustrative and not intended to be restrictive.

The specific meanings of the abbreviations used in this invention are as follows:
Aib: α-amino isobutyric
α-MeF: α-methyl phenylalanine
α-MeF (2F): α-methyl-2-fluorophenylalanine
α-MeL: α-methyl leucine
Ac3c: 1-aminocyclopropanecarboxylic acid
AEEAc: [2-(2-amino ethoxy)-ethoxy]-acetyl
cAMP: Adenosine Cyclophosphate
Fmoc: fluorene methoxycarbonyl
Boc: tert butoxycarbonyl
DMF: Dimethylformamide
HBTU: Benzotriazol-N, N, N', N'-Tetramethylurea hexafluorophosphate
Trt: Triphenylmethyl
ivdde: 1-(4,4-dimethyl-2,6-dioxocyclohexyl)-3-methyl-butyl
tBu: tert butyl
OtBu: oxygen tert butyl
TFA: trifluoroacetic acid
Tis: triisopropylsilane
HPLC/MS: High performance liquid chromatography/mass spectrometry
HPLC-UV: High Performance Liquid Chromatography UV
IPTG: isopropyl-β-D-thiogalactoside
Tris: Trihydroxymethylaminomethane
DCM: Dichloromethane
THF: Tetrahydrofuran
DIPEA: N, N-diisopropylethylamine
NMP: N-methylpyrrolidone
HEK-293: Human embryonic kidney cells
CHO: Chinese Hamster Ovarian Cells
GLP-1: Glucagon like peptide-1
GIP: Glucose-dependent insulinotropic polypeptide
GCG: Glucagon
GLP-1 R: Glucagon like peptide-1 receptor
GIP R: Glucose-dependent insulinotropic polypeptide receptor
GCG R: Glucagon receptor
NAFLD: Nonalcoholic fatty liver disease
NASH: Nonalcoholic steatohepatitis
DMEM: Du's improved Eagle medium
FBS: Fetal bovine serum
FCS: calf serum
P/S: Penicillin/Streptomycin
PBS: Phosphate buffer solution
HBSS: Hank's buffer salt solution
EC₅₀: Half effect concentration
IBMX: 3-isobutyl-1-methylxanthine.

### DETAILED DESCRIPTION

The following embodiments and experimental examples are provided to further illustrate the present application, but, in no way, limit the effective scope of the present application.

### Example 1: synthesis of peptide compounds

The intermediates and compounds of the present application can be synthesized and prepared through various methods known in the art. The following specific embodiments illustrate the use of chemical synthesis methods to prepare the compounds of the present application. Each specific synthesis step described can be combined using different materials and methods to synthesize various corresponding compounds or salts of the present application. The reagents and raw materials used are easily accessible to ordinary technical personnel in this field. Specifically, the following embodiments are only intended to illustrate the present application and should not limit the scope of the present application in any way.

### Material:

The materials and reagents used in the present application are all purchased from commercial commodities, and the protective amino acids used in the entire synthesis process are as follows: Fmoc-Ser(tBu)-OH, Fmoc-Ac3c-OH, Fmoc-Pro-OH, Fmoc-Ala-OH, Fmoc-Gly-OH, Fmoc-Glu(OtBu)-OH, Fmoc-Leu-OH, Fmoc-Gln(Trt)-OH, Fmoc-Ile-OH, Fmoc-Phe-OH, Fmoc-Asp(OtBu)-OH, Fmoc-Lys(Boc)-OH, Fmoc-Lys(ivdde)-OH, Fmoc-α-MeLeu-OH, Fmoc-Thr(tBu)-OH, Fmoc-His(Trt)-OH, Fmoc-α-MePhe-OH, Fmoc-α-MePhe(2F)-OH, Fmoc-Aib-OH, Boc-Tyr(tBu)-OH.

The synthesis and preparation method of the compound of the present application is illustrated below taking Compound 10 as an example (where the preparation of other compounds only requires changing the synthesis order of amino acid raw materials).
(1) Rink Amide MBHA resin pre-treatment: 1g of dried Rink Amide MBHA resin (substitution degree S=0.28mmol/g) was weighted, and added into a reaction column, added with 10ml of DMF, and blown with nitrogen gas for 30min. The solvent was removed, another 10ml of DMF was added to wash for 3 times, with 1 minute for each time, and the solvent was removed.
(2) Removal of protective group Fmoc: 20% piperidine/DMF solution (10ml) was added to the treated Rink amino resin, and was left to react for 20min under nitrogen atmosphere, during which the degree of reaction was monitored using the ninhydrin colorimetric method, and when the resin color shows blue, it indicated successful removal of Fmoc. filtering was performed to remove the solvent after the reaction, DMF was added to the reaction system to wash the resin for 1 minute, which was repeated for 6 times.
(3) Coupling reaction (peptide bond formation): the corresponding Fmoc protected amino acid solution (3.0eq) was added into the reactor, then added with DIEA (6.0eq), added with 5ml of DMF to the reaction column, blown with nitrogen gas, and subjected to HBTU (2.85eq) after amino acid dissolution. The nitrogen gas was adjusted to evenly bubble the resin to react at 25 °C for 30 minutes, during which the degree of reaction was monitored by using the ninhydrin colorimetric method, and, when the resin was colorless and transparent, it indicated successful coupling. After the reaction is completed, filtering was performed to remove the solvent. DMF was added to the reaction system, the resin was stirred and washed for 1 minute, which was repeated for 6 times. The above steps were repeated and corresponding amino acid solutions were added in turn until the peptide chain synthesis was completed. The last amino acid was coupled with Boc-Tyr(tBu)-OH, and the coupling was judged to be completed by detecting the colorless and transparent resin with tetrachlorobenzoquinone. The Lys of the side chain modification site was replaced with Fmoc-Lys(ivdde)-OH. The sequence of adding amino acid coupling in the synthesis of Compound 10 main peptide sequence was Fmoc-Ser(tBu)-OH, Fmoc-Ac3c-OH, Fmoc-Pro-OH (2x), Fmoc-Ala-OH, Fmoc-Gly-OH, Fmoc-Ser(tBu)-OH (2x), Fmoc-Pro-OH, Fmoc-Gly-OH (2x), Fmoc-Glu(OtBu)-OH, Fmoc-Leu-OH (2x), Fmoc-Phe-OH, Fmoc-Glu(OtBu)-OH, Fmoc-Ile-OH, Fmoc-Phe-OH, Fmoc-Ala-OH, Fmoc-Aib-OH, Fmoc-Gln(Trt)-OH, Fmoc-Ala-OH, Fmoc-Lys(ivdde)-OH, Fmoc-Lys(Boc)-OH, Fmoc-Asp(OtBu)-OH, Fmoc-Leu-OH, Fmoc-Phe-OH, Fmoc-Ile-OH, Fmoc-Ser(tBu)-OH, Fmoc-Tyr(tBu)-OH, Fmoc-Asp(OtBu)-OH , Fmoc-Ser(tBu)-OH, Fmoc-Thr(tBu)-OH, Fmoc-α-MeF(2F)-OH, Fmoc-Thr(tBu)-OH, Fmoc-Gly-OH, Fmoc-Gln(Trt)-OH, Fmoc-Aib-OH, Boc-Tyr(tBu)-OH.
(4) Removal of the side chain protection group ivdde from Lys: 3% hydrazine hydrate/DMF solution (10ml) was added into the reaction column, and left to react under nitrogen gas for 20 minutes to remove the side chain protection group ivdde from the modified site Lys. The blue color of the ninhydrin detection resin indicated complete removal. After successful removal, the solvent was removed, DMF was added to the system to wash for 1 minute, and the solvent was removed, in which washing was performed for 6 times.
(5) Lys side chain modification: AEEAc (2.0eq) was added to the above resin. DIEA (4.00eq) was added, and 5ml of DMF was added to the reaction column. It was blown with nitrogen gas and added with HBTU (1.9eq) after the amino acids were dissolved. The nitrogen gas was adjusted to evenly bubble the resin to react at 25 °C for 1 hour. The ninhydrin detection resin was colorless and transparent, indicating a complete reaction. The reaction solution was removed, and washed for 6 times with DMF (10ml), with 1 minute for each time. 20% piperidine/DMF (10ml) was added to the reaction column, and blown with nitrogen gas for 20 minutes to remove Fmoc groups. DMF (10ml) was added to wash for 6 times, with 1 minute for each time, and the solvent was removed. According to the above steps AEEAc, Fmoc-Glu(OtBu)-OH, and C₂₀ monotert butyl ester were added successively to conduct coupling reaction, so as to complete the side chain modification. Finally, the resin was shrunk with MeOH (10ml) for 3 minutes each time, the solvent was evacuated, and the resin was poured out and dried, ready for use.
(6) Post treatment of peptide resin: the above dried peptide resin was added with prepared cutting reagent (95% TFA: 2.5% Ti: 2.5% H₂O), and shaken in a shaking table for 2.5 hours to cut the resin. Filtering was performed to obtain a filtrate, which was added to 10 times the volume of ice isopropyl ether, centrifuged, and washed for 5 times with isopropyl ether. Crude peptides were obtained by vacuum drying for 2 hours.
(7) Purification of crude peptide compounds:

The obtained peptide powder was dissolved in a 50% acetonitrile/H₂O solution and purified using a reverse phase C18 preparative column (Shimadzu, Inertsil ODS 20x 250 mm 5um). Using 95% buffer A (0.1% TFA/H₂O) and 5% buffer B (0.075% TFA/acetonitrile) as the initial eluent and gradually increasing the proportion of buffer B to 75%, the purification was run continuously for 30 minutes for elution and collecting target peptide components. The purified peptide compound was analyzed and confirmed by analytical HPLC/MS method. The purity of the obtained peptide compound was not less than 95%.

**Table 1. List of Synthetic Peptide Compounds and Their Molecular Weights**

| Compound | Sequence | Theoretical MW | Observed MW |
|---|---|---|---|
| Semaglutide | H-Aib-EGTFTSDVSSYLEGQAAK(AEEAc-A EEAc-γGlu-CO(CH2)16COOH)EFIAWLVRGR G-OH | 4113.64 | 4113.6 |
| Tirzepatide | Y-Aib-QGTFTSDYSI-Aib-LDKIAQK(AEEAc -AEEAc-γGlu-CO(CH2)18COOH)AFVQWLIA GGPSSGAPPPS-NH2 | 4814.32 | 4815.6 |
| 1 (SEQ ID NO: 1) | Y-Aib-QGT-αMeF-TSDYSI-αMeL-LDKK(AE EAc-AEEAc-γGlu-CO(CH2)18COOH)AQ-Aib- AFIEYLLETGPSSGAPP-Ac3c-S-NH2 | 4920.56 | 4920.6 |
| 2 (SEQ ID NO:2) | Y-Aib-QGT-αMeF-TSDYSI-αMeL-LDKK(AE EAc-AEEAc-γGlu-CO(CH2)18COOH)AQ-Aib- AFIEYLLETHPSSGAPP-Ac3c-S-NH2 | 5000.64 | 5000.7 |
| 3 (SEQ ID NO:3) | Y-Aib-QGT-αMeF-TSDYSI-αMeL-LDKK(AE EAc-AEEAc-γGlu-CO(CH2)18COOH)AQ-Aib- AFIEYLLESHPSSGAPP-Ac3c-S-NH2 | 4986.62 | 4987.2 |
| 4 (SEQ ID NO:4) | Y-Aib-QGT-αMeF(2F)-TSDYSIFLDKK(AEE Ac-AEEAc-γGlu-CO(CH2)18COOH)AQ-Aib-A FIEYLLEGGPSSGAPP-Ac3c-S-NH2 | 4914.48 | 4914.3 |
| 5 (SEQ ID NO:5) | Y-Aib-QGT-αMeF(2F)-TSDYSIFLDKK(AEE Ac-AEEAc-γGlu-CO(CH2)18COOH)AQ-Aib-A FIEFLLEGGPSSGAPP-Ac3c-S-NH2 | 4898.48 | 4898.4 |
| 6 (SEQ ID NO6:) | Y-Aib-HGT-αMeF(2F)-TSDYSI-αMeL-LDKK (AEEAc-AEEAc-γGlu-CO(CH2)18COOH)AQ- Aib-AFIEYLLE –Aib-HPSSGQPPPS-NH2 | 5082.72 | 5082.9 |
| 7 (SEQ ID NO:7) | Y-Aib-QGT-αMeF-TSDYSI-αMeL-LDKK(AE EAc-AEEAc-γGlu-CO(CH2)18COOH)AQ-Aib- AFIEYLLETGPSSG-Aib-PPPS-NH2 | 4948.61 | 4948.8 |
| 8 (SEQ ID NO:8) | Y-Aib-QGT-αMeF-TSDYSI-αMeL-LDKK(AE EAc-AEEAc-γGlu-CO(CH2)18COOH)AQ-Aib- AFIEYLLETGPSSGQPPPS-NH2 | 4991.63 | 4991.7 |
| 9 (SEQ ID NO:9) | Y-Aib-QGT-αMeF(2F)-TSDYSIFLDKK(AEE Ac-AEEAc-γGlu-CO(CH2)18COOH)AQ-Aib-A FIEFLLETGPSSGAPP-Ac3c-S-NH2 | 4942.54 | 4943.1 |
| 10 (SEQ ID NO:10) | Y-Aib-QGT-αMeF(2F)-TSDYSIFLDKK(AEE Ac-AEEAc-γGlu-CO(CH₂)₁₈COOH)AQ-Aib-A FIEFLLESHPSSGAPP-Ac3c-S-NH₂ | 5008.60 | 5008.4 |
| 11 (SEQ ID NO:11) | Y-Aib-QGT-αMeF(2F)-TSDYSIFLDKK(AEE Ac-AEEAc-γGlu-CO(CH₂)₁₈COOH)AQ-Aib-A FIEFLLETGPSSGAPP-Ac3c-S-NH₂ | 4924.55 | 4924.2 |
| 12 (SEQ ID NO:12) | Y-Aib-QGT-αMeF-TSDYSIFLDKK(AEEAc- AEEAc-γGlu-CO(CH2)18COOH)AQ-Aib-AFIE FLLETGPSSGAPPPS-NH2 | 4938.57 | 4939.5 |
| 13 (SEQ ID NO:13) | Y-Aib-QGT-αMeF-TSDYSIFLDKK(AEEAc- AEEAc-γGlu-CO(CH2)18COOH)AQ-Aib-AFIE YLLETGPSSGAPPPS-NH2 | 4954.57 | 4955.1 |
| 14 (SEQ ID NO:14) | Y-Aib-QGT-αMeF-TSDYSI-αMeL-LDKK(AE EAc-AEEAc-γGlu-CO(CH2)18COOH)AQ-Aib- AFIEFLLETGPSSGAPPPS-NH2 | 4918.58 | 4918.8 |
| 15 (SEQ ID NO:15) | Y-Aib-QGT-αMeF-TSDYSIFLDKK(AEEAc- AEEAc-γGlu-CO(CH2)18COOH)AQ-Aib-AFIE FLLESHPSSGAPPPS-NH2 | 5004.63 | 5004.6 |
| 16 (SEQ ID NO:16) | Y-Aib-QGT-αMeF-TSDYSIFLDKK(AEEAc- AEEAc-γGlu-CO(CH2)18COOH)AQ-Aib-AFIE FLLE-Aib-HPSSGAPPPS-NH2 | 5002.66 | 5002.2 |
| 17 (SEQ ID NO:17) | Y-Aib-QGT-αMeF(2F)-TSDYSIFLDKK(AEE Ac-AEEAc-γGlu-CO(CH2)18COOH)AQ-Aib-A FIEYLLETGPSSGAPPPS-NH2 | 4972.56 | 4972.2 |
| 18 (SEQ ID NO:18) | Y-Aib-QGT-αMeF(2F)-TSDYSIFLDKK(AEE Ac-AEEAc-γGlu-CO(CH2)18COOH)AQ-Aib-A FIEYLLE-Aib-HPSSGAPPPS-NH2 | 5036.65 | 5036.7 |
| 19 (SEQ ID NO:19) | Y-Aib-QGT-αMeF(2F)-TSDYSI-αMeL-LDKK (AEEAc-AEEAc-γGlu-CO(CH2)18COOH)AQ- Aib-AFIEFLLETGPSSGAPPPS-NH2 | 4936.57 | 4937.1 |
| 20 (SEQ ID NO:20) | Y-Aib-QGT-αMeF(2F)-TSDYSI-αMeL-LDKK (AEEAc-AEEAc-γGlu-CO(CH₂)₁₈COOH)AQ- Aib-AFIEFLLE-Aib-HPSSGAPPPS-NH₂ | 5000.66 | 5000.7 |
| 21 (SEQ ID NO:21) | Y-Aib-QGT-αMeF(2F)-TSDYSIFLDKK(AEE Ac-AEEAc-γGlu-CO(CH2)18COOH)AQ-Aib-A FIEFLLEGGPSSGQPPPS-NH2 | 4985.56 | 4985.7 |
| 22 (SEQ ID NO:22) | Y-Aib-QGT-αMeF(2F)-TSDYSI-αMeL-LDKK (AEEAc-AEEAc-γGlu-CO(CH2)18COOH)AQ- Aib-AFIEFLLEGGPSSGQPPPS-NH2 | 4949.57 | 4950 |
| 23 (SEQ ID NO:23) | Y-Aib-QGT-αMeF(2F)-TSDYSI-αMeL-LDKK (AEEAc-AEEAc-γGlu-CO(CH2)18COOH)AQ- Aib-AFIEYLLETGPSSGQPPPS-NH2 | 5009.62 | 5009.7 |
| 24 (SEQ ID NO:24) | Y-Aib-QGT-αMeF(2F)-TSDYSI-αMeL-LDKK (AEEAc-AEEAc-γGlu-CO(CH2)18COOH)AQ- Aib-AFIEFLLETGPSS-Aib-HPPPS-NH2 | 4986.64 | 4986.9 |
| 25 (SEQ ID NO:25) | Y-Aib-QGT-αMeF(2F)-TSDYSI-αMeL-LDKK (AEEAc-AEEAc-γGlu-CO(CH2)18COOH)AQ- Aib-AFIEFLLEGRPSS-Aib-HPPPS-NH2 | 5085.77 | 5085.6 |
| 26 (SEQ ID NO:26) | Y-Aib-QGT-αMeF-TSDYSI-αMeL-LDKK(AE EAc-AEEAc-γGlu-CO(CH2)18COOH)AQ-Aib- AFIEFLLESHPSSGAPPPS-NH2 | 4984.65 | 4984.2 |
| 27 (SEQ ID NO:27) | Y-Aib-QGT-αMeF-TSDYSI-αMeF-LDKK(AE EAc-AEEAc-γGlu-CO(CH2)18COOH)AQ-Aib- AFIEYLLESHPSSGAPPPS-NH2 | 5034.66 | 5034.9 |
| 28 (SEQ ID NO:28) | Y-Aib-QGT-αMeF-TSDYSI-αMeF-LDKK(AE EAc-AEEAc-γGlu-CO(CH2)18COOH)AQ-Aib- AFIEFLLES-Aib-PSSGAPP-Ac3c-S-NH2 | 4918.58 | 4919.1 |
| 29 (SEQ ID NO:29) | Y-Aib-QGT-αMeF-TSDYSIFLDKK(AEEAc- AEEAc-γGlu-CO(CH2)18COOH)AQ-Aib-AFIE YLLES-Aib-PSSGAPP-Ac3c-S-NH2 | 4954.57 | 4954.5 |
| 30 (SEQ ID NO:30) | Y-Aib-QGT-αMeF-TSDYSI-αMeL-LDKK(AE EAc-AEEAc-γGlu-CO(CH2)18COOH)AQ-Aib- AFIEFLLET-Aib-PSSGAPP-Ac3c-S-NH2 | 4932.61 | 4932.6 |
| 31 (SEQ ID NO:31) | Y-Aib-QGT-αMeF(2F)-TSDYSI-αMeL-LDKK (AEEAc-AEEAc-γGlu-CO(CH2)18COOH)AQ- Aib-AFIEFLLE-Aib-HPSSGAPP-A c3c-S-NH2 | 4986.64 | 4986.6 |
| 32 (SEQ ID NO:32) | Y-Aib-QGT-αMeF(2F)-TSDYSIFLDKK(AEE Ac-AEEAc-γGlu-CO(CH2)18COOH)AQ-Aib-A FIEYLLE-Aib-HPSSGAPP-Ac3c-S-NH2 | 5022.63 | 5022.9 |
| 33 (SEQ ID NO:33) | Y-Aib-QGT-αMeF-TSDYSI-αMeL-LDKK(AE EAc-AEEAc-γGlu-CO(CH2)18COOH)AQ-Aib- AFIEFLLESHPSS-Aib-HPP-Ac3c-S-NH2 | 5064.73 | 5064.8 |
| 34 (SEQ ID NO:34) | Y-Aib-QGT-αMeF(2F)-TSDYSI-αMeL-LDKK (AEEAc-AEEAc-γGlu-CO(CH2)18COOH)AQ- Aib-AFIEYLLETGPSSGQPP-Ac3c-S-NH2 | 4995.60 | 4996.2 |
| 35 (SEQ ID NO:35) | Y-Aib-QGT-αMeF-TSDYSI-αMeL-LDKK(AE EAc-AEEAc-γGlu-CO(CH2)18COOH)AQ-Aib- AFIEYLLETGPSSGQPP-Ac3c-S-NH2 | 4977.61 | 4977.6 |
| 36 (SEQ ID NO:36) | Y-Aib-QGT-αMeF-TSDYSI-αMeL-LDKK(AE EAc-AEEAc-γGlu-CO(CH2)18COOH)AQ-Aib- AFIEFLLEGGPSS-Aib-HPPPS-NH2 | 4968.65 | 4968.9 |
| 37 (SEQ ID NO:27) | Y-Aib-QGT-αMeF-TSDYSI-αMeL-LDKK(AE EAc-AEEAc-γGlu-CO(CH2)18COOH)AQ-Aib- AFIEFLLEGRPSS-Aib-HPPPS-NH2 | 5067.78 | 5068.0 |
| 38 (SEQ ID NO:38) | Y-Aib-QGT-αMeF(2F)-TSDYSI-αMeL-LDKK (AEEAc-AEEAc-γGlu-CO(CH2)18COOH)AQ- Aib-AFIEFLLETGPSS-Aib-HPPPS-NH2 | 5030.69 | 5030.8 |
| 39 (SEQ ID NO:39) | Y-Aib-QGT-αMeF(2F)-TSDYSI-αMeL-LDKK (AEEAc-AEEAc-γGlu-CO(CH2)18COOH)AQ- Aib-AFIEFLLETRPSS-Aib-HPPPS-NH2 | 5129.82 | 5130.0 |
| 40 (SEQ ID NO:40) | Y-Aib-QGT-αMeF(2F)-TSDYSI-αMeL-LDKK (AEEAc-AEEAc-γGlu-CO(CH2)18COOH)AQ- Aib-AFIEFLLESHPSS-Aib-HPP-Ac3c-S-NH2 | 5082.72 | 5082.8 |
| 41 (SEQ ID NO:41) | Y-Aib-QGT-αMeF(2F)-TSDYSILLDKK(AEE Ac-AEEAc-γGlu-CO(CH2)18COOH)AQ-Aib-A FIEFLLESHPSS-Aib-HPP-Ac3c-S-NH2 | 5068.70 | 5068.8 |
| 42 (SEQ ID NO:42) | Y-Aib-QGT-αMeF-TSDYSILLDKK(AEEAc- AEEAc-γGlu-CO(CH2)18COOH)AQ-Aib-AFIE FLLESHPSS-Aib-HPP-Ac3c-S-NH2 | 5050.71 | 5050.8 |
| 43 (SEQ ID NO:43) | Y-Aib-QGT-αMeF(2F)-TSDYSI-αMeL-LDKK (AEEAc-AEEAc-γGlu-CO(CH2)18COOH)AQ- Aib-AFIEYLLESHPSS-Aib-HPP-Ac3c-S-NH2 | 5098.72 | 5098.8 |
| 44 (SEQ ID NO:44) | Y-Aib-QGTFTSDYSI-αMeL-LDKK(AEEAc- AEEAc-γGlu-CO(CH2)18COOH)AQ-Aib-AFIE YLLEGGPSSGAPPPS-NH2 | 4876.50 | 4876.5 |
| 45 (SEQ ID NO:45) | Y-Aib-QGTFTSDYSI-αMeL-LDKK(AEEAc- AEEAc-γGlu-CO(CH2)18COOH)AQ-Aib-AFIE YLLE-Aib-GPSSGAPPPS-NH2 | 4759.40 | 4759.5 |

### Example 2: Activity Test of Peptide Compounds on Stable Transgenic Cells of Human GLP-1/GIP/GCG Receptors

Firstly, HEK-293 cells stably overexpressing human GLP-1 or GCG receptors and CHO cells stably overexpressing GIP receptors were constructed separately. By measuring the cAMP signal response levels of the aforementioned cells, the agonistic activity of individual compound towards the corresponding receptor was determined. The intracellular cAMP content was measured using reagent kit available from Cisbio Corp. based on HTRF (homogeneous time-resolved fluorescence) technology.

Frozen cells stably overexpressing human GLP-1, GIP, or GCG receptors was placed in a 37 °C constant temperature water bath for rapid thawing and recovery. The cell fluid was transferred to 10ml of HBSS for resuspension, and centrifuged at 1000 rpm for 5 minutes at room temperature. The supernatant was discarded, and cells were re-suspended in 1 × HBSS (containing 0.1% Casein, 250 µM IBMX), and cell density was adjusted to 1.0 × 10⁵/mL.

10 µL cell suspension was added to each well in the 384 well plate. The compound to be tested was dissolved in 1 × PBS buffer to prepare a stock solution in, which was diluted step by step at 3 times to prepare a total of 12-concentration-point compound solutions. Using the ECHO liquid transfer system, 100nL of the prepared compound solution was added to the corresponding cell suspensions in the 384 well plate, rotated and shaken at 1000rpm for 1 minute to mix evenly, and then incubated at room temperature for 60 minutes. 10 µL of detection reagent was added to each well after drug incubation was completed, and incubation was performed in the kit at room temperature for 60 minutes. The plate was placed in the EnVision multifunctional enzyme reader (PerkinElmer) and the reading was acquired at 665/615 nm. GraphPad Prism 5 plotting software was used to create compound concentration response curves and EC₅₀ (nM) values was calculated.

Using natural wild-type human GLP-1 (7-37), GIP, and GCG as positive controls for the receptor activation effect of the tested compound, for GLP-1 receptor cells, the relative activity (%) of the tested compound was evaluated by calculating a ratio of the EC₅₀ value of human GLP-1 (7-37) to the EC₅₀ value of the tested compound.

For GIP receptor cells, the relative activity (%) of the tested compound was evaluated by calculating the percentage of the ratio of the EC₅₀ value of human GIP to the EC₅₀ value of the tested compound.

For GCG receptor cells, the relative activity (%) of the tested compound was evaluated by calculating the percentage of the EC₅₀ value of human GCG to the EC₅₀ value of the tested compound.

**Table 2. Stable cell activity of human GLP-1/GIP/GCG receptors for peptide compounds**

| | Human GLP-1/GIP/GCGR stable transfected cell cAMP activity | | | | | |
|---|---|---|---|---|---|---|
| Compound | Human GLP-1R | | Human GIP R | | Human GCG R | |
| | EC₅₀ (nM) | Rel. A (%) | EC₅₀, (nM) | Rel. A (%) | EC₅₀ (nM) | Rel. A (%) |
| GLP-1(7-37) | 0.044 | 100.00 | NT | - | NT | - |
| GIP | NT | - | 0.181 | 100.00 | NT | - |
| GCG | NT | - | NT | - | 0.057 | 100.00 |
| Semaglutide | 0.021 | 209.52 | NT | - | NT | - |
| Tirzepatide | 0.179 | 24.58 | 0.210 | 86.19 | NT | - |
| 1 | 0.021 | 209.52 | 0.015 | 1206.67 | 0.099 | 57.58 |
| 2 | 0.026 | 169.23 | 0.025 | 724.00 | 0.232 | 24.57 |
| 3 | 0.044 | 100.00 | 0.024 | 754.17 | 0.120 | 47.50 |
| 4 | 0.041 | 107.32 | 0.057 | 317.54 | 0.071 | 80.28 |
| 5 | 0.022 | 200.00 | 0.049 | 369.39 | 0.070 | 81.43 |
| 6 | 0.020 | 220.00 | 0.014 | 1292.86 | 0.058 | 98.28 |
| 7 | 0.010 | 440.00 | 0.013 | 1392.31 | 0.058 | 98.28 |
| 8 | 0.029 | 151.72 | 0.012 | 1508.33 | 0.071 | 80.28 |
| 9 | 0.017 | 258.82 | 0.047 | 385.11 | 0.062 | 91.94 |
| 10 | 0.047 | 93.62 | 0.021 | 861.90 | 0.076 | 75.00 |
| 11 | 0.010 | 440.00 | 0.085 | 212.94 | 0.104 | 54.81 |
| 12 | 0.014 | 314.29 | 0.082 | 220.73 | 0.417 | 13.67 |
| 13 | 0.012 | 366.67 | 0.032 | 565.63 | 0.052 | 109.62 |
| 14 | 0.010 | 440.00 | 0.011 | 1645.45 | 0.114 | 50.00 |
| 15 | 0.023 | 191.30 | 0.033 | 548.48 | 0.139 | 41.01 |
| 16 | 0.020 | 220.00 | 0.054 | 335.19 | 0.168 | 33.93 |
| 17 | 0.011 | 400.00 | 0.019 | 952.63 | 0.035 | 162.86 |
| 18 | 0.012 | 366.67 | 0.021 | 861.90 | 0.026 | 219.23 |
| 19 | 0.009 | 488.89 | 0.008 | 2262.50 | 0.029 | 196.55 |
| 20 | 0.010 | 440.00 | 0.006 | 3016.67 | 0.023 | 247.83 |
| 21 | 0.005 | 880.00 | 0.008 | 2262.50 | 0.025 | 228.00 |
| 22 | 0.006 | 733.33 | 0.007 | 2585.71 | 0.017 | 335.29 |
| 23 | 0.005 | 880.00 | 0.007 | 2585.71 | 0.025 | 228.00 |
| 24 | 0.005 | 880.00 | 0.007 | 2585.71 | 0.026 | 219.23 |
| 25 | 0.031 | 141.94 | 0.013 | 1392.31 | 0.034 | 167.65 |
| 26 | 0.022 | 200.00 | 0.007 | 2585.71 | 0.130 | 43.85 |
| 27 | 0.016 | 275.00 | 0.023 | 786.96 | 0.059 | 96.61 |
| 28 | 0.024 | 183.33 | 0.011 | 1645.45 | 0.154 | 37.01 |
| 29 | 0.016 | 275.00 | 0.309 | 58.58 | 0.069 | 82.61 |
| 30 | 0.020 | 220.00 | 0.015 | 1206.67 | 0.173 | 32.95 |
| 31 | 0.012 | 366.67 | 0.009 | 2011.11 | 0.027 | 211.11 |
| 32 | 0.014 | 314.29 | 0.011 | 1645.45 | 0.017 | 335.29 |
| 33 | 0.019 | 231.58 | 0.008 | 2262.50 | 0.155 | 36.77 |
| 34 | 0.023 | 191.30 | 0.005 | 3620.00 | 0.038 | 150.00 |
| 35 | 0.022 | 200.00 | 0.008 | 2262.50 | 0.087 | 65.52 |
| 36 | 0.025 | 176.00 | 0.007 | 2585.71 | 0.045 | 126.67 |
| 37 | 0.028 | 157.14 | 0.015 | 1206.67 | 0.082 | 69.51 |
| 38 | 0.038 | 115.79 | 0.013 | 1392.31 | 0.068 | 83.82 |
| 39 | 0.078 | 56.41 | 0.015 | 1206.67 | 0.153 | 37.25 |
| 40 | 0.016 | 275.00 | 0.120 | 150.83 | 0.167 | 34.13 |
| 41 | 0.012 | 366.67 | 0.073 | 247.95 | 0.081 | 70.37 |
| 42 | 0.009 | 488.89 | 0.070 | 258.57 | 0.495 | 11.52 |
| 43 | 0.010 | 440.00 | 0.041 | 441.46 | 0.127 | 44.88 |

| | | | | | | |
|---|---|---|---|---|---|---|
| Rel.A: Relative Activity; NT: Not test, not tested; Each group is subjected to at least 3 independent tests. | | | | | | |

The data in the table shows that all compounds can exhibit high relative activity in human GLP-1/GIP/GCG receptor stable cells.

### Example 3: Functional Activity Test of Peptide Compounds on Rat Islet Tumor RIN-m5F Cells

The RIN-m5F cell line derived from rat pancreatic islet tissue mainly expresses endogenous GLP-1/GIP/GCG receptors, with GLP-1 receptors being the most abundant. This experiment measured the cAMP level produced by the compound on RIN-m5F cells.

Frozen RIN-m5F cells were placed in a 37 °C constant temperature water bath for rapid thawing and recovery. The cell solution was transferred to 10ml of HBSS for resuspension, and centrifuged at 1000 rpm for 5 minutes at room temperature. The supernatant was discarded, and cells were resuspended in 1 × HBSS (containing 0.1% Casein, 250 µM IBMX), and cell density was adjusted to 1.0 × 10⁵/mL.

10 µL cell suspension was added to each well in the 384 well plate. The compound to be tested was dissolved in 1 × PBS buffer to prepare a stock solution, which was diluted step by step by 3 times to prepare a total of 12-concentration-point compound solutions. Using the ECHO liquid transfer system, 100nL of the prepared compound solution was added to the corresponding cell suspensions in the 384 well plate, rotated and shaken at 1000rpm for 1 minute to mix evenly, and then incubated at room temperature for 60 minutes. 10 µL of detection reagent was added to each wells after drug incubation was completed, and incubation was performed for 60 minutes. The plate was placed in the EnVision multifunctional enzyme reader (PerkinElmer) and the reading was acquired at 665/615 nm. GraphPad Prism 5 plotting software was used to create compound concentration-effect curves and calculation was performed.

**Table 3. cell activity of peptide compounds on RIN-m5F rat pancreatic islet tumor**

| Compound | EC₅₀(nM) | Rel.A (%) |
|---|---|---|
| GLP-1 (7-3 7) | 1.032 | 100.00 |
| Semaglutide | 0.223 | 462.78 |
| Tirzepatide | 2.846 | 36.26 |
| 1 | 0.467 | 220.99 |
| 2 | 0.624 | 165.38 |
| 3 | 0.400 | 258.00 |
| 4 | 0.275 | 375.27 |
| 5 | 0.929 | 111.09 |
| 6 | 1.594 | 64.74 |
| 7 | 1.344 | 76.79 |
| 8 | 0.771 | 133.85 |
| 9 | 0.322 | 320.50 |
| 10 | 0.274 | 376.64 |
| 13 | 0.164 | 629.27 |
| 14 | 0.239 | 431.80 |
| 15 | 0.694 | 148.70 |
| 17 | 0.122 | 845.90 |
| 18 | 0.214 | 482.24 |
| 19 | 0.149 | 692.62 |
| 20 | 0.267 | 386.52 |
| 21 | 0.191 | 540.31 |
| 22 | 0.119 | 867.23 |
| 23 | 0.134 | 770.15 |
| 24 | 0.096 | 1075.00 |
| 25 | 0.202 | 510.89 |
| 26 | 0.401 | 257.36 |
| 27 | 0.252 | 409.52 |
| 28 | 0.784 | 131.63 |
| 29 | 0.633 | 163.03 |
| 30 | 0.878 | 117.54 |
| 31 | 0.274 | 376.64 |
| 32 | 0.208 | 496.15 |
| 33 | 0.717 | 143.93 |
| 34 | 0.879 | 117.41 |
| 35 | 0.346 | 298.27 |
| 36 | 0.200 | 516.00 |
| 37 | 0.176 | 586.36 |
| 38 | 0.173 | 596.53 |
| 39 | 0.843 | 122.42 |
| 40 | 0.160 | 645.00 |
| 41 | 0.136 | 758.82 |
| 42 | 0.359 | 287.47 |
| 43 | 0.260 | 396.92 |

| | | |
|---|---|---|
| Rel.A: Relative Activity; NT: Not test, not tested; Each group was subjected to at least 3 independent tests. | | |

The compounds of the present application in the table can exhibit high relative activity in pancreatic islet tumor cells RIN-m5F.

### Example 4: Functional activity test of peptide compounds on 3T3-L1 adipocytes

Mouse 3T3-L1 precursor adipocytes can be induced to differentiate into mature adipocytes. 3T3-L1 cells to was directly inoculated by a cell density of 4×10⁴/ml into a 96 well plate and cultured in DMEM medium containing 10% FCS and 1% P/S, and incubated at 37 °C in a 5% CO2 incubator. After the cells became full and contacted with each other, DMEM differentiation medium containing 20% FBS, 0.5 mM IBMX, 0.4 ug/mL dexamethasone, and 5 ug/mL insulin was used to induce differentiation of 3T3-L1 adipocyte. After 5 days of induction and cultivation, DMEM medium containing 20% FBS, 4 ug/ml insulin, and 10 uM rosiglitazone was used to continue induction and cultivation for 3 days. Then, DMEM medium containing 10% FBS was used to continue cultivation for 2-3 days to induce mature 3T3-L1 adipocytes.

Induced differentiation and maturation of 3T3-L1 adipocytes, similar to adipose tissue cells, can express abundant GIP receptors. The cAMP level produced by the compound on 3T3-L1 adipocytes was determined in this experiment. The tested compound solution was diluted step by step according to a concentration gradient of 3 times, to obtain a total of 10 series of concentration solutions. The solutions were added to induced mature 3T3-L1 adipocytes and incubated at room temperature for 60 minutes. 10µL detection reagent in the kit was added to each well after drug incubation, and incubated at room temperature for another 60 minutes. The plate was placed in the EnVision multifunctional enzyme reader (PerkinElmer) to measure the reading at 665/615 nm. GraphPad Prism 5 plotting software was used to create compound concentration-effect curves and calculation was performed.

**Table 4. Activity of peptide compounds Mouse on 3T3-L1 adipocyte**

| Compound | EC₅₀(nM) | Rel.A (%) |
|---|---|---|
| GIP | 3.802 | 100 |
| Semaglutide | NT | - |
| Tirzepatide | 87.932 | 4.32 |
| 1 | 7.221 | 52.65 |
| 2 | 2.491 | 152.63 |
| 3 | 1.869 | 203.42 |
| 4 | 5.402 | 70.38 |
| 5 | 3.747 | 101.47 |
| 6 | 1.015 | 374.58 |
| 7 | 4.299 | 88.44 |
| 8 | 3.660 | 103.88 |
| 9 | 19.752 | 19.25 |
| 10 | 6.156 | 61.76 |
| 11 | 17.905 | 21.23 |
| 12 | 5.974 | 63.64 |
| 13 | 10.912 | 34.84 |
| 17 | 9.730 | 39.08 |
| 18 | 1.987 | 191.34 |
| 19 | 3.010 | 126.31 |
| 20 | 0.447 | 850.56 |
| 21 | 2.189 | 173.69 |
| 22 | 0.475 | 800.42 |
| 23 | 2.451 | 155.12 |
| 24 | 0.401 | 948.13 |
| 25 | 0.918 | 414.16 |
| 26 | 1.808 | 210.29 |
| 27 | 6.524 | 58.28 |
| 28 | 9.848 | 38.61 |
| 29 | 20.652 | 18.41 |
| 30 | 7.084 | 53.67 |
| 31 | 0.677 | 561.60 |
| 32 | 1.106 | 343.76 |
| 33 | 3.300 | 115.21 |
| 34 | 2.297 | 165.52 |
| 35 | 6.008 | 63.28 |
| 36 | 1.017 | 373.84 |
| 37 | 2.487 | 152.87 |
| 38 | 3.238 | 117.42 |
| 39 | 2.046 | 185.83 |
| 40 | 2.561 | 148.46 |
| 41 | 1.508 | 252.12 |
| 42 | 2.732 | 139.17 |
| 43 | 1.358 | 279.97 |
| 44 | 10.608 | 35.84 |
| 45 | 8.108 | 46.89 |

| | | |
|---|---|---|
| Rel. A: Relative Activity; NT: Not test, not tested; Each group was subjected to at least 3 independent tests. | | |

The compounds of the present application in the table can exhibit high relative activity on adipocytes 3T3-L1.

### Example 5: Functional activity test of peptide compounds on human primary liver cells

The cAMP levels produced by compounds on human primary liver cells were determined in this experiment. Human primary liver cells were purchased from Lonza (cell product number HUCPG).

Frozen human primary liver cells were placed in a 37 °C constant temperature water bath for rapid thawing and recovery. The cell solution was transferred to 10ml of HBSS for resuspension, and centrifuged at 1000 rpm for 5 minutes at room temperature. The supernatant was discarded, and cells were resuspended in 1 × HBSS (containing 0.1% Casein, 250 µM IBMX), and cell density was adjusted to 1.0 × 10⁵/mL.

10µL cell suspension was added to each well in the 384 well plate. The compound to be tested was dissolved in 1 × PBS buffer to prepare a stock solution, which was diluted step by step by 3 times to prepare a total of 12-concentration-point compound solutions. Using the ECHO liquid transfer system, 100nL of the prepared compound solution was added to the corresponding cell suspensions in the 384 well plate, rotated and shaken at 1000rpm for 1 minute to mix evenly, and then incubated at room temperature for 60 minutes. 10 µL of detection reagent was added to each well after drug incubation was completed, and incubation was performed for 60 minutes. The plate was placed in the EnVision multifunctional enzyme reader (PerkinElmer) and the reading was acquired at 665/615 nm. GraphPad Prism 5 plotting software was used to create compound concentration-effect curves and calculation was performed.

**Table 5. Activity of peptide compounds on human primary liver cells**

| Compound | EC₅₀(nM) | Rel.A (%) |
|---|---|---|
| GCG | 3.891 | 100 |
| Semaglutide | >500 | - |
| Tirzepatide | NT | - |
| 1 | 1.648 | 236.10 |
| 2 | 2.368 | 164.32 |
| 3 | 1.589 | 244.87 |
| 4 | 0.957 | 406.58 |
| 5 | 0.649 | 599.54 |
| 6 | 0.299 | 1301.34 |
| 7 | 1.949 | 199.64 |
| 8 | 0.995 | 391.06 |
| 9 | 3.976 | 97.86 |
| 10 | 5.188 | 75.00 |
| 13 | 4.501 | 86.45 |
| 14 | 4.566 | 85.22 |
| 15 | 7.216 | 53.92 |
| 17 | 1.679 | 231.75 |
| 18 | 0.622 | 625.56 |
| 19 | 0.971 | 400.72 |
| 20 | 0.846 | 459.93 |
| 21 | 0.497 | 782.90 |
| 22 | 0.654 | 594.95 |
| 23 | 1.238 | 314.30 |
| 24 | 0.280 | 1389.64 |
| 25 | 0.649 | 599.54 |
| 26 | 2.621 | 148.45 |
| 27 | 9.721 | 40.03 |
| 28 | 5.206 | 74.74 |
| 29 | 2.928 | 132.89 |
| 30 | 7.825 | 49.73 |
| 31 | 0.454 | 857.05 |
| 32 | 0.580 | 670.86 |
| 33 | 4.187 | 92.93 |
| 34 | 0.537 | 724.58 |
| 35 | 1.960 | 198.52 |
| 36 | 0.612 | 635.78 |
| 37 | 1.043 | 373.06 |
| 38 | 0.899 | 432.81 |
| 39 | 2.029 | 191.77 |
| 40 | 1.068 | 364.33 |
| 41 | 0.907 | 429.00 |
| 42 | 1.364 | 285.26 |
| 43 | 0.463 | 840.39 |
| 44 | 7.785 | 49.98 |

| | | |
|---|---|---|
| Rel. A: Relative Activity; NT: Not test, not tested; Each group was subjected to at least 3 independent tests. | | |

The compounds of the present application in the table can exhibit high relative activity on liver cells.

### Example 6: In vivo pharmacodynamics

High-fat diet induced obesity (DIO) mice have characteristics such as obesity, elevated blood sugar, insulin resistance, and lipid abnormalities, all of which are very significant metabolic syndromes similar to a human body. The effects of the compounds of the present application on body weight, food intake, blood sugar, and lipids in C57 BL/6J DIO mice were studied.

5-week-old male C57 BL/6J mice (purchased from Shanghai Slake Experimental Animal Company) were raised in a pathogen free and clean environment (at a temperature of 20-24 °C and relative humidity of 30-70%), with 12 hours of light/12 hours of dark circulation. They were fed on normal feed, with 4 animals per cage and an adapting period of 2 weeks. Obesity was induced in mice by feeding with a high-fat diet (60 kcal% of calories from fat). After 16 weeks of feeding on a high-fat diet, the weight of DIO mice reached 41-55g, and a blood glucose range reached 8-12mmol/L. DIO mice were randomly divided into groups (n=6 /group) according to their weight and fasting blood glucose, so that each group of animals had a similar average body weight and blood glucose. After grouping, one animal was raised in each cage for one week, during which each of the animals was injected subcutaneously (S.C.) with a solvent (1 × PBS, 5 ml/kg) to pre-adapt the experimental operation process.

After the preparation of animals, vehicle control or compounds were administered to groups of animals by subcutaneous injection, in which the compounds were dissolved in 1 × PBS, the dosage was 5 ml/kg, and the administration operation was started at 9:00 am and conducted once every three days (Q3D) for 15 days. Throughout the entire experimental study, animal weight and food intake were measured daily before administration, and compared with initial weight and food intake of the same animal before administration to calculate a percentage change in animal weight (%) and cumulative food intake, so as to evaluate the impact of compounds on changes in body weight and food intake.

At the end point of the experiment (Day 15), the body weight of the mice was measured, and then fasting blood glucose was measured by taking blood from the tail tip of broken tail without anesthesia. After the blood collection, the animals were anesthetized with CO₂ and euthanized. Then, blood was taken from the heart and plasma was separated by centrifugation. The plasma was used to measure total cholesterol (TC), low-density lipoprotein cholesterol (LDL-C), triglycerides (TG), and blood insulin content. The liver was separated for homogenization, and then centrifuged to obtain the supernatant for determining triglyceride content in the liver.

All results were expressed in Mean ± SEM, and the results were analyzed by one- way ANOVA using GraphPad Prism 5 software, followed by Dunnett's post hoc test compared to the Vehicle control group. The difference at the p<0.05 level was considered statistically significant.

**Table 6-1. Effects of compounds on body weight changes in DIO mice**

| **Compound** | **Dosage (nmol/kg)** | **Weight change (%)** |
|---|---|---|
| **Vehicle** | **-** | -2.6±1.78 |
| **Tirzepatide** | **3** | -16.1±0.71 |
| | **10** | -21.6±0.99 |
| | **30** | -32.6±1.60 |
| **3** | **3** | -21.2±0.75 |
| | **10** | -32.4±2.94 |
| | **30** | -51.6±1.21 |
| **9** | **3** | -17.6±1.51 |
| | **10** | -21.5±1.02 |
| | **30** | -35.4±3.61 |
| **18** | **3** | -22.2±0.89 |
| | **10** | -30.6±1.26 |
| | **30** | -45.0±1.52 |
| **22** | **3** | -20.3±1.54 |
| | **10** | -28.9±2.28 |
| | **30** | -50.5±1.45 |

**Table 6-2. Effects of compounds on body weight changes in DIO mice**

| **Compound** | **Dosage (nmol/kg)** | **Weight change (%)** |
|---|---|---|
| **Vehicle** | - | 2.7±0.77 |
| **17** | **3** | -13.6±0.90 |
| | **10** | -22.7±1.87 |
| **38** | **3** | -19.0±1.30 |
| | **10** | -25.7±2.26 |

From the results in Tables 6-1 and 6-2, it can be seen that the compound of the present application has a significant effect on weight reduction.

**Table 7-1. Effects of compounds on blood lipids in DIO mice**

| **Compound** | **Dosage (nmol/kg)** | **TG (mM)** | **TC (mM)** | **LDL-C (mM)** |
|---|---|---|---|---|
| **Vehicle** | - | 1.24±0.074 | 5.52±0.430 | 0.92±0.121 |
| **Tirzepatide** | **3** | 0.97±0.050 | 4.12±0.322 | 0.62±0.065 |
| | **30** | 0.79±0.068 | 3.28±0.259 | 0.57±0.057 |
| **3** | **3** | 0.87±0.066 | 3.42±0.214 | 0.52±0.033 |
| | **30** | 0.55±0.022 | 2.15±0.079 | 0.37±0.035 |
| **9** | **3** | 0.92±0.063 | 4.00±0.179 | 0.59±0.024 |
| | **30** | 0.70±0.023 | 2.72±0.106 | 0.51±0.028 |
| **18** | **3** | 0.90±0.027 | 3.77±0.234 | 0.60±0.043 |
| | **30** | 0.61±0.041 | 2.32±0.161 | 0.35±0.036 |
| **22** | **3** | 0.84±0.051 | 3.77±0.353 | 0.63±0.070 |
| | **30** | 0.59±0.075 | 2.46±0.076 | 0.48±0.047 |

**Table 7-2. Effects of compounds on blood lipids in DIO mice**

| **Compound** | **Dosage(nmol/kg)** | **TG (mM)** | **TC (mM)** | **LDL-C(mM)** |
|---|---|---|---|---|
| **Vehicle** | **-** | 0.95±0.078 | 4.91±0.360 | 0.44±0.041 |
| **17** | **3** | 0.82±0.015 | 3.85±0.174 | 0.43±0.033 |
| | **10** | 0.64±0.040 | 3.19±0.216 | 0.38±0.024 |
| **38** | **3** | 0.74±0.019 | 3.49±0.151 | 0.39±0.025 |
| | **10** | 0.63±0.051 | 3.12±0.186 | 0.35±0.053 |

From the results in Tables 7-1 and 7-2, it can be seen that the compound of the present application has a significant therapeutic effect on reducing blood lipid.

**Table 8-1. Effects of 3nM compounds on blood glucose and insulin levels in DIO mice**

| **Compound** | **Dosage (nmol/kg)** | **Blood sugar (mg/dL)** | **Insulin (mIU/L)** |
|---|---|---|---|
| **Vehicle** | **-** | 156.5±8.48 | 63.0±14.71 |
| **3** | **3** | 107.5±5.17 | 25.4±16.90 |
| **9** | **3** | 97.9±4.07 | 25.2±6.14 |
| **18** | **3** | 98.5±9.17 | 21.2±3.73 |
| **22** | **3** | 99.4±5.65 | 24.7±7.45 |

**Table 8-2. Effects of 3nM compounds on blood glucose and insulin levels in DIO mice**

| **Compound** | **Dosage (nmol/kg)** | **Blood sugar (mg/dL)** | **Insulin (mIU/L)** |
|---|---|---|---|
| **Vehicle** | - | 210.8±6.23 | 72.2±12.29 |
| **17** | **3** | 141.1±2.45 | 37.5±18.49 |
| **19** | **3** | 142.0±2.92 | 26.1±4.77 |
| **38** | **3** | 151.7±8.46 | 24.9±5.43 |

From the results in Tables 8-1 and 8-2, it can be seen that the compound of the present application has a significant therapeutic effect on reducing blood sugar and blood insulin.

**Table 9-1. Effects of 3nM compounds on the liver TG of DIO mice**

| **Compound** | **Dosage (nmol/kg)** | **Liver TG (mg)** |
|---|---|---|
| **Vehicle** | **-** | 181.5±33.45 |
| **3** | **3** | 32.2±2.99 |
| **10** | **3** | 81.6±8.50 |
| **20** | **3** | 26.7±2.81 |
| **24** | **3** | 30.7±1.31 |

**Table 9-2. Effects of 3nM compounds on the liver TG of DIO mice**

| **Compound** | **Dosage (nmol/kg)** | **Liver TG (mg)** |
|---|---|---|
| **Vehicle** | **-** | 129.8±20.21 |
| **19** | **3** | 38.0±3.37 |
| **21** | **3** | 41.4±6.33 |
| **40** | **3** | 42.1±5.22 |

From the results in Tables 9-1 and 9-2, it can be seen that the compound of the present application has a significant therapeutic effect on reducing liver triglycerides.

### Example 7: Rat Pharmacokinetics (PK) Study of Compounds

7~9 weeks old male SD rats (220-250g, 3 rats/group) were subjected to subcutaneous injection of 30nmol/kg compound. After administration, blood was collected through the jugular vein at time points 0.25, 2, 4, 8, 12, 16, 24, 48, 96, 120, and 144 hours. The blood was processed to obtain plasma samples and analyzed using LC-MS/MS. The blood concentration time curve was analyzed using Phoenix WinNonlin version 6.3 software (non compartment model), and PK parameters and half-life were calculated.

The PK parameters calculated using the above method are shown in Table 10.

**Table 10 Pharmacokinetic (PK) parameters of compounds in rats**

| **Compound** | **Cₘₐₓ (nmol/L)** | **Tₘₐₓ (h)** | **T_{1/2} (h)** | **AUC₀₋ₗₐₛₜ (nmol·h/L)** |
|---|---|---|---|---|
| **3** | 148.9 | 21.3 | 19.1 | 6687.2 |
| **17** | 136.0 | 17.3 | 17.3 | 5808.1 |
| **18** | 62.8 | 16.0 | 15.6 | 3007.4 |
| **38** | 78.1 | 16.0 | 15.7 | 3098.4 |

Table 10 shows that individual compounds exhibit an extended pharmacokinetic distribution.

## Claims

1. GLP-1/GIP/GCG R triple agonist polypeptide compounds or salts or solvates thereof with general formula (I):
Y-Aib-X3-GT-X6-TSDYSI-X13-LDK-X17-AQ-Aib-AFIE-X25-LLE-X29-X30-PSS-X34 -X35-PP-X38- S-R¹ (I)
wherein,
X3 is Q or H;
X6 is F α MeF, or α MeF (2F);
X13 is α MeL, F, α MeF, or L;
X17 is ψ;
X25 is Y or F;
X29 is T, S, G, or Aib;
X30 is G, H, R, or Aib;
X34 is G or Aib;
X35 is A, Q, Aib, or H;
X38 is Ac3c or P;
R¹ is NH₂ or OH, or pharmaceutically acceptable salts and/or esters thereof;
where, ψ is Lys with side chains modified by the following general formula (II):
Y-Z (II),
wherein
Y is (AEEAc or Glu)ₐ-(AEEAc or Glu)b-(AEEAc or Glu)_{c}, wherein a, b, and c are independently 0 or 1 separately, and a, b, and c are not zero at the same time, and a carboxyl end of Y is connected to ε-amino of side chain of Lys ; and
Z is-CO-(CH₂) ₘ-R², m is an integer between 6 and 24, and R² is selected from-COOH.

2. The polypeptide compound or salts or solvates thereof according to claim 1, **characterized in that**, the compound of general formula (I) comprises at least two specific amino acids at the following sites:
X13 is F or α MeF;
X25 is F;
X29 is T or S;
X30 is H, R, or Aib;
X35 is Q, Aib, or H;
X38 is Ac3c.

3. The polypeptide compound or salts or solvates thereof according to claim 1, **characterized in that**, the general formula (II) is AEEAc-AEEAc-γGlu-CO(CH₂)₁₈COOH.

4. The polypeptide compound or salts or solvates thereof according to claim 1, **characterized in that** the compound is selected from:
| Compound | Sequence |
|---|---|
| 1 | Y-Aib-QGT-αMeF-TSDYSI-αMeL-LDKK(AEEAc-AEEAc-γGlu-CO(CH2)18C OOH)AQ-Aib-AFIEYLLETGPSSGAPP-Ac3c-S-NH2 |
| 2 | Y-Aib-QGT-αMeF-TSDYSI-αMeL-LDKK(AEEAc-AEEAc-γGlu-CO(CH2)18C OOH)AQ-Aib-AFIEYLLETHPSSGAPP-Ac3c-S-NH2 |
| 3 | Y-Aib-QGT-αMeF-TSDYSI-αMeL-LDKK(AEEAc-AEEAc-γGlu-CO(CH2)18C OOH)AQ-Aib-AFIEYLLESHPSSGAPP-Ac3c-S-NH2 |
| 4 | Y-Aib-QGT-αMeF(2F)-TSDYSIFLDKK(AEEAc-AEEAc-γGlu-CO(CH2)18CO OH)AQ-Aib-AFIEYLLEGGPSSGAPP-Ac3c-S-NH2 |
| 5 | Y-Aib-QGT-αMeF(2F)-TSDYSIFLDKK(AEEAc-AEEAc-γGlu-CO(CH₂)₁₈CO OH)AQ-Aib-AFIEFLLEGGPSSGAPP-Ac3c-S-NH₂ |
| 6 | Y-Aib-HGT-αMeF(2F)-TSDYSI-αMeL-LDKK(AEEAc-AEEAc-γGlu-CO(CH2 )18COOH)AQ-Aib-AFIEYLLE –Aib-HPSSGQPPPS-NH2 |
| 7 | Y-Aib-QGT-αMeF-TSDYSI-αMeL-LDKK(AEEAc-AEEAc-γGlu-CO(CH₂)₁₈C OOH)AQ-Aib-AFIEYLLETGPSSG-Aib-PPPS-NH₂ |
| 8 | Y-Aib-QGT-αMeF-TSDYSI-αMeL-LDKK(AEEAc-AEEAc-γGlu-CO(CH₂)₁₈C OOH)AQ-Aib-AFIEYLLETGPSSGQPPPS-NH₂ |
| 9 | Y-Aib-QGT-αMeF(2F)-TSDYSIFLDKK(AEEAc-AEEAc-γGlu-CO(CH₂)₁₈CO OH)AQ-Aib-AFIEFLLETGPSSGAPP-Ac3c-S-NH₂ |
| 10 | Y-Aib-QGT-αMeF(2F)-TSDYSIFLDKK(AEEAc-AEEAc-γGlu-CO(CH₂)₁₈CO OH)AQ-Aib-AFIEFLLESHPSSGAPP-Ac3c-S-NH₂ |
| 11 | Y-Aib-QGT-αMeF(2F)-TSDYSIFLDKK(AEEAc-AEEAc-γGlu-CO(CH2)18CO OH)AQ-Aib-AFIEFLLETGPSSGAPP-Ac3c-S-NH2 |
| 12 | Y-Aib-QGT-αMeF-TSDYSIFLDKK(AEEAc-AEEAc-γGlu-CO(CH2)18COOH) AQ-Aib-AFIEFLLETGPSSGAPPPS-NH2 |
| 13 | Y-Aib-QGT-αMeF-TSDYSIFLDKK(AEEAc-AEEAc-γGlu-CO(CH2)18COOH) AQ-Aib-AFIEYLLETGPSSGAPPPS-NH2 |
| 14 | Y-Aib-QGT-αMeF-TSDYSI-αMeL-LDKK(AEEAc-AEEAc-γGlu-CO(CH2)18C OOH)AQ-Aib-AFIEFLLETGPSSGAPPPS-NH2 |
| 15 | Y-Aib-QGT-αMeF-TSDYSIFLDKK(AEEAc-AEEAc-γGlu-CO(CH2)18COOH) AQ-Aib-AFIEFLLESHPSSGAPPPS-NH2 |
| 16 | Y-Aib-QGT-αMeF-TSDYSIFLDKK(AEEAc-AEEAc-γGlu-CO(CH2)18COOH) AQ-Aib-AFIEFLLE-Aib-HPSSGAPPPS-NH2 |
| 17 | Y-Aib-QGT-αMeF(2F)-TSDYSIFLDKK(AEEAc-AEEAc-γGlu-CO(CH2)18CO OH)AQ-Aib-AFIEYLLETGPSSGAPPPS-NH2 |
| 18 | Y-Aib-QGT-αMeF(2F)-TSDYSIFLDKK(AEEAc-AEEAc-γGlu-CO(CH₂)₁₈CO OH)AQ-Aib-AFIEYLLE-Aib-HPSSGAPPPS-NH₂ |
| 19 | Y-Aib-QGT-αMeF(2F)-TSDYSI-αMeL-LDKK(AEEAc-AEEAc-γGlu-CO(CH2 )18COOH)AQ-Aib-AFIEFLLETGPSSGAPPPS-NH2 |
| 20 | Y-Aib-QGT-αMeF(2F)-TSDYSI-αMeL-LDKK(AEEAc-AEEAc-γGlu-CO(CH2 )18COOH)AQ-Aib-AFIEFLLE-Aib-HPSSGAPPPS-NH2 |
| 21 | Y-Aib-QGT-αMeF(2F)-TSDYSIFLDKK(AEEAc-AEEAc-γGlu-CO(CH2)18CO OH)AQ-Aib-AFIEFLLEGGPSSGQPPPS-NH2 |
| 22 | Y-Aib-QGT-αMeF(2F)-TSDYSI-αMeL-LDKK(AEEAc-AEEAc-γGlu-CO(CH2 )18COOH)AQ-Aib-AFIEFLLEGGPSSGQPPPS-NH2 |
| 23 | Y-Aib-QGT-αMeF(2F)-TSDYSI-αMeL-LDKK(AEEAc-AEEAc-γGlu-CO(CH2 )18COOH)AQ-Aib-AFIEYLLETGPSSGQPPPS-NH2 |
| 24 | Y-Aib-QGT-αMeF(2F)-TSDYSI-αMeL-LDKK(AEEAc-AEEAc-γGlu-CO(CH2 )18COOH)AQ-Aib-AFIEFLLETGPSS-Aib-HPPPS-NH2 |
| 25 | Y-Aib-QGT-αMeF(2F)-TSDYSI-αMeL-LDKK(AEEAc-AEEAc-γGlu-CO(CH2 )18COOH)AQ-Aib-AFIEFLLEGRPSS-Aib-HPPPS-NH2 |
| 26 | Y-Aib-QGT-αMeF-TSDYSI-αMeL-LDKK(AEEAc-AEEAc-γGlu-CO(CH₂)₁₈C OOH)AQ-Aib-AFIEFLLESHPSSGAPPPS-NH₂ |
| 27 | Y-Aib-QGT-αMeF-TSDYSI-αMeF-LDKK(AEEAc-AEEAc-γGlu-CO(CH₂)₁₈C OOH)AQ-Aib-AFIEYLLESHPSSGAPPPS-NH₂ |
| 28 | Y-Aib-QGT-αMeF-TSDYSI-αMeF-LDKK(AEEAc-AEEAc-γGlu-CO(CH₂)₁₈C OOH)AQ-Aib-AFIEFLLES-Aib-PSSGAPP-Ac3c-S-NH₂ |
| 29 | Y-Aib-QGT-αMeF-TSDYSIFLDKK(AEEAc-AEEAc-γGlu-CO(CH2)18COOH) AQ-Aib-AFIEYLLES-Aib-PSSGAPP-Ac3c-S-NH2 |
| 30 | Y-Aib-QGT-αMeF-TSDYSI-αMeL-LDKK(AEEAc-AEEAc-γGlu-CO(CH2)18C OOH)AQ-Aib-AFIEFLLET-Aib-PSSGAPP-Ac3c-S-NH2 |
| 31 | Y-Aib-QGT-αMeF(2F)-TSDYSI-αMeL-LDKK(AEEAc-AEEAc-γGlu-CO(CH2 )18COOH)AQ-Aib-AFIEFLLE-Aib-HPSSGAPP-Ac3c-S-NH2 |
| 32 | Y-Aib-QGT-αMeF(2F)-TSDYSIFLDKK(AEEAc-AEEAc-γGlu-CO(CH2)18CO OH)AQ-Aib-AFIEYLLE-Aib-HPSSGAPP-Ac3c-S-NH2 |
| 33 | Y-Aib-QGT-αMeF-TSDYSI-αMeL-LDKK(AEEAc-AEEAc-γGlu-CO(CH₂)₁₈C OOH)AQ-Aib-AFIEFLLESHPSS-Aib-HPP-Ac3c-S-NH₂ |
| 34 | Y-Aib-QGT-αMeF(2F)-TSDYSI-αMeL-LDKK(AEEAc-AEEAc-γGlu-CO(CH2 )18COOH)AQ-Aib-AFIEYLLETGPSSGQPP-Ac3c-S-NH2 |
| 35 | Y-Aib-QGT-αMeF-TSDYSI-αMeL-LDKK(AEEAc-AEEAc-γGlu-CO(CH2)18C OOH)AQ-Aib-AFIEYLLETGPSSGQPP-Ac3c-S-NH2 |
| 36 | Y-Aib-QGT-αMeF-TSDYSI-αMeL-LDKK(AEEAc-AEEAc-γGlu-CO(CH2)18C OOH)AQ-Aib-AFIEFLLEGGPSS-Aib-HPPPS-NH2 |
| 37 | Y-Aib-QGT-αMeF-TSDYSI-αMeL-LDKK(AEEAc-AEEAc-γGlu-CO(CH₂)₁₈C OOH)AQ-Aib-AFIEFLLEGRPSS-Aib-HPPPS-NH₂ |
| 38 | Y-Aib-QGT-αMeF(2F)-TSDYSI-αMeL-LDKK(AEEAc-AEEAc-γGlu-CO(CH2 )18COOH)AQ-Aib-AFIEFLLETGPSS-Aib-HPPPS-NH2 |
| 39 | Y-Aib-QGT-αMeF(2F)-TSDYSI-αMeL-LDKK(AEEAc-AEEAc-γGlu-CO(CH2 )18COOH)AQ-Aib-AFIEFLLETRPSS-Aib-HPPPS-NH2 |
| 40 | Y-Aib-QGT-αMeF(2F)-TSDYSI-αMeL-LDKK(AEEAc-AEEAc-γGlu-CO(CH2 )18COOH)AQ-Aib-AFIEFLLESHPSS-Aib-HPP-Ac3c-S-NH2 |
| 41 | Y-Aib-QGT-αMeF(2F)-TSDYSILLDKK(AEEAc-AEEAc-γGlu-CO(CH2)18CO OH)AQ-Aib-AFIEFLLESHPSS-Aib-HPP-Ac3c-S-NH2 |
| 42 | Y-Aib-QGT-αMeF-TSDYSILLDKK(AEEAc-AEEAc-γGlu-CO(CH2)18COOH) AQ-Aib-AFIEFLLESHPSS-Aib-HPP-Ac3c-S-NH2 |
| 43 | Y-Aib-QGT-αMeF(2F)-TSDYSI-αMeL-LDKK(AEEAc-AEEAc-γGlu-CO(CH2 )18COOH)AQ-Aib-AFIEYLLESHPSS-Aib-HPP-Ac3c-S-NH2 |

5. The polypeptide compound or salts or solvates thereof according to claim 4, **characterized in that**, the compound is further selected from:
| Compound | Sequence |
|---|---|
| 2 | Y-Aib-QGT-αMeF-TSDYSI-αMeL-LDKK(AEEAc-AEEAc-γGlu-CO(C H2)18COOH)AQ-Aib-AFIEYLLETHPSSGAPP-Ac3c-S-NH2 |
| 3 | Y-Aib-QGT-αMeF-TSDYSI-αMeL-LDKK(AEEAc-AEEAc-γGlu-CO(C H2)18COOH)AQ-Aib-AFIEYLLESHPSSGAPP-Ac3c-S-NH2 |
| 5 | Y-Aib-QGT-αMeF(2F)-TSDYSIFLDKK(AEEAc-AEEAc-γGlu-CO(CH2 )18COOH)AQ-Aib-AFIEFLLEGGPSSGAPP-Ac3c-S-NH2 |
| 8 | Y-Aib-QGT-αMeF-TSDYSI-αMeL-LDKK(AEEAc-AEEAc-γGlu-CO(C H2)18COOH)AQ-Aib-AFIEYLLETGPSSGQPPPS-NH2 |
| 18 | Y-Aib-QGT-αMeF(2F)-TSDYSIFLDKK(AEEAc-AEEAc-γGlu-CO(CH2 )18COOH)AQ-Aib-AFIEYLLE-Aib-HPSSGAPPPS-NH2 |
| 19 | Y-Aib-QGT-αMeF(2F)-TSDYSI-αMeL-LDKK(AEEAc-AEEAc-γGlu-C O(CH₂)₁₈COOH)AQ-Aib-AFIEFLLETGPSSGAPPPS-NH₂ |
| 20 | Y-Aib-QGT-αMeF(2F)-TSDYSI-αMeL-LDKK(AEEAc-AEEAc-γGlu-C O(CH2)18COOH)AQ-Aib-AFIEFLLE-Aib-HPSSGAPPPS-NH2 |
| 21 | Y-Aib-QGT-αMeF(2F)-TSDYSIFLDKK(AEEAc-AEEAc-γGlu-CO(CH2 )18COOH)AQ-Aib-AFIEFLLEGGPSSGQPPPS-NH2 |
| 22 | Y-Aib-QGT-αMeF(2F)-TSDYSI-αMeL-LDKK(AEEAc-AEEAc-γGlu-C O(CH2)18COOH)AQ-Aib-AFIEFLLEGGPSSGQPPPS-NH2 |
| 23 | Y-Aib-QGT-αMeF(2F)-TSDYSI-αMeL-LDKK(AEEAc-AEEAc-γGlu-C O(CH2)18COOH)AQ-Aib-AFIEYLLETGPSSGQPPPS-NH2 |
| 24 | Y-Aib-QGT-αMeF(2F)-TSDYSI-αMeL-LDKK(AEEAc-AEEAc-γGlu-C O(CH₂)₁₈COOH)AQ-Aib-AFIEFLLETGPSS-Aib-HPPPS-NH₂ |
| 25 | Y-Aib-QGT-αMeF(2F)-TSDYSI-αMeL-LDKK(AEEAc-AEEAc-γGlu-C O(CH₂)₁₈COOH)AQ-Aib-AFIEFLLEGRPSS-Aib-HPPPS-NH₂ |
| 26 | Y-Aib-QGT-αMeF-TSDYSI-αMeL-LDKK(AEEAc-AEEAc-γGlu-CO(C H2)18COOH)AQ-Aib-AFIEFLLESHPSSGAPPPS-NH2 |
| 31 | Y-Aib-QGT-αMeF(2F)-TSDYSI-αMeL-LDKK(AEEAc-AEEAc-γGlu-C O(CH2)18COOH)AQ-Aib-AFIEFLLE-Aib-HPSSGAPP-Ac3c-S-NH2 |
| 32 | Y-Aib-QGT-αMeF(2F)-TSDYSIFLDKK(AEEAc-AEEAc-γGlu-CO(CH2 )18COOH)AQ-Aib-AFIEYLLE-Aib-HPSSGAPP-Ac3c-S-NH2 |
| 33 | Y-Aib-QGT-αMeF-TSDYSI-αMeL-LDKK(AEEAc-AEEAc-γGlu-CO(C H2)18COOH)AQ-Aib-AFIEFLLESHPSS-Aib-HPP-Ac3c-S-NH2 |
| 34 | Y-Aib-QGT-αMeF(2F)-TSDYSI-αMeL-LDKK(AEEAc-AEEAc-γGlu-C O(CH2)18COOH)AQ-Aib-AFIEYLLETGPSSGQPP-Ac3c-S-NH2 |
| 36 | Y-Aib-QGT-αMeF-TSDYSI-αMeL-LDKK(AEEAc-AEEAc-γGlu-CO(C H2)18COOH)AQ-Aib-AFIEFLLEGGPSS-Aib-HPPPS-NH2 |
| 37 | Y-Aib-QGT-αMeF-TSDYSI-αMeL-LDKK(AEEAc-AEEAc-γGlu-CO(C H2)18COOH)AQ-Aib-AFIEFLLEGRPSS-Aib-HPPPS-NH2 |
| 38 | Y-Aib-QGT-αMeF(2F)-TSDYSI-αMeL-LDKK(AEEAc-AEEAc-γGlu-C O(CH₂)₁₈COOH)AQ-Aib-AFIEFLLETGPSS-Aib-HPPPS-NH₂ |
| 39 | Y-Aib-QGT-αMeF(2F)-TSDYSI-αMeL-LDKK(AEEAc-AEEAc-γGlu-C O(CH₂)₁₈COOH)AQ-Aib-AFIEFLLETRPSS-Aib-HPPPS-NH₂ |
| 40 | Y-Aib-QGT-αMeF(2F)-TSDYSI-αMeL-LDKK(AEEAc-AEEAc-γGlu-C O(CH2)18COOH)AQ-Aib-AFIEFLLESHPSS-Aib-HPP-Ac3c-S-NH2 |
| 41 | Y-Aib-QGT-αMeF(2F)-TSDYSILLDKK(AEEAc-AEEAc-γGlu-CO(CH2 )18COOH)AQ-Aib-AFIEFLLESHPSS-Aib-HPP-Ac3c-S-NH2 |
| 42 | Y-Aib-QGT-αMeF-TSDYSILLDKK(AEEAc-AEEAc-γGlu-CO(CH2)18C OOH)AQ-Aib-AFIEFLLESHPSS-Aib-HPP-Ac3c-S-NH2 |
| 43 | Y-Aib-QGT-αMeF(2F)-TSDYSI-αMeL-LDKK(AEEAc-AEEAc-γGlu-C O(CH2)18COOH)AQ-Aib-AFIEYLLESHPSS-Aib-HPP-Ac3c-S-NH2 |

6. The polypeptide compound or salts or solvates thereof according to claim 1, **characterized in that**, in terms of activation ability of GLP-1 receptor stable transfected cells, compared to natural GLP-1, the compound has a relative activity of at least 30%, preferably at least 60%, more preferably at least 80%, and further preferably at least 100% in terms of activation ability of GLP-1 receptor.

7. The polypeptide compound or salts or solvates thereof according to claim 1, **characterized in that**, in terms of activation ability of GIP receptor stable transfected cell, compared to natural GIP, the compound has a relative activity of at least 100% and more preferably at least 150% in terms of activation ability of GIP receptor.

8. The polypeptide compound or salts or solvates thereof according to claim 1, **characterized in that**, in terms of activation ability of GCG receptor stable transfected cells, compared to natural GCG, the compound has a relative activity of at least 10%, more preferably at least 30% in terms of activation ability of GCG receptor.

9. The polypeptide compound or salts or solvates thereof according to claim 1, **characterized in that**, in terms of the representative cell RIN-m5F of pancreatic islet tissue, compared to natural GLP-1 (7-37), the compound has a relative activity of at least 60%, preferably at least 80%, and more preferably at least 100% in terms of activation ability of RIN-m5F cell.

10. The polypeptide compound or salts or solvates thereof according to claim 1, **characterized in that**, in terms of a representative cell 3T3-L1 of adipose tissue, compared to natural GIP, the compound has a relative activity of at least 60%, and preferably at least 100% in terms of activation ability of 3T3-L1 cell.

11. The polypeptide compound or salts or solvates thereof according to claim 1, **characterized in that**, in terms of human primary liver cells representative of liver tissue, compared to natural GCG, the compound has a relative activity of at least 60%, and preferably at least 100% in terms of activation ability of liver cells.

12. A pharmaceutical composition, comprising an effective amount of any of the compounds or salts or solvates thereof according to claims 1-11, and pharmaceutically acceptable excipients, diluents, carriers, or excipients.

13. The pharmaceutical composition according to claim 12, **characterized in that**, the pharmaceutical composition is injection, lyophilized powder, tablet, pill, pastille, soft capsule, hard capsule, granule, powder, solution, suspension or syrup; and alternatively, the drug composition is in the form of microcapsule, microspheres, nanoparticle, or liposome.

14. The pharmaceutical composition according to claim 12, **characterized in that**, the pharmaceutical composition is used for oral administration, inhalation administration, or parenteral administration, and the parenteral administration is selected from intraperitoneal, intramuscular, arterial, intravenous, subcutaneous, or intradermal injection administration.

15. The pharmaceutical composition according to claim 12, **characterized in that**, the pharmaceutical composition is administered at a frequency of at least once a day, once a week, once two weeks, or once a month.

16. The pharmaceutical composition according to claim 12, **characterized in that**, the pharmaceutical composition is used in combination with at least one therapeutic active substance, and the therapeutic active substance comprises anti-diabetes activator, GIP receptor agonist, GCG receptor agonist or antagonist, GLP-1/GIP receptor agonist, GLP-1/GCG receptor agonist, GIP/GCG receptor agonist, FGF-21 and analogues thereof, cholecystokinin B (CCKB) and analogues thereof PYY (3-36) and analogues thereof, leptin and analogues thereof, calcitonin and analogues thereof, lipid-regulating active drugs, PPAR-α, β, δ Agonists or regulators, antiplatelet aggregation activators, PCSK9 inhibitors, lipase inhibitors, anti-fibrotic or cirrhotic activators, or anti-inflammatory activators.

17. The pharmaceutical composition according to claim 16, **characterized in that**, the anti-diabetes activator comprises insulin and analogues thereof, biguanides, sulfonylureas, thiazolidinediones, α-Glucosidase inhibitors, DPP-4 inhibitors, SGLT2 inhibitors, dual SGLT1/SGLT2 inhibitors, GLP-1 receptor agonists, or amylin and analogues thereof.

18. Use of the compounds or salts or solvates thereof according to any one of claims 1-11 or the pharmaceutical combination according to any one of claims 12-17 in the preparation of drugs for promoting insulin secretion and lowering blood sugar.

19. Use of the compounds or salts or solvates thereof according to any one of claims 1-11 or the pharmaceutical combination according to any one of claims 12-17 in the preparation of drugs for inhibiting ingestion, delaying gastric emptying, increasing energy consumption, and reducing body weight.

20. Use of the compounds or salts or solvates thereof according to any one of claims 1-11 or the pharmaceutical combination according to any one of claims 12-17 in the preparation of drugs for reducing pancreatic islets β-cell apoptosis, increasing pancreatic islets β-cell number, and improving the function of pancreatic islet cells.

21. Use of the compounds or salts or solvates thereof according to any one of claims 1-11 or the pharmaceutical combination according to any one of claims 12-17 in the preparation of drugs for improving blood lipids, reducing liver fat accumulation, inhibiting the development of liver inflammation, and preventing and treating non-alcoholic fatty liver disease.

22. Use of the compounds or salts or solvates thereof according to any one of claims 1-11 or the pharmaceutical combination according to any one of claims 12-17 in the preparation of drugs for promoting the growth of brain neurons, eliminating neurotoxic substances, inhibiting inflammation development, and exerting neuroprotective effects.

23. Use of the compounds or salts or solvates thereof according to any one of claims 1-11 or the pharmaceutical combination according to any one of claims 12-17 in the preparation of drugs for the prevention and/or treatment of metabolic disorders and related complications, preferably for the treatment of diabetes, obesity or non-alcoholic fatty liver disease.

24. Use of the compounds or salts or solvates thereof according to any one of claims 1-11 or the pharmaceutical combination according to any one of claims 12-17 in the preparation of drugs for treating dyslipidemia and related diseases, and neurodegenerative diseases whichinclude Parkinson's disease and Alzheimer's disease.

25. Use of the compounds or salts or solvates thereof according to any one of claims 1-11 or the pharmaceutical combination according to any one of claims 12-17 in the preparation of drugs for treating bone diseases related to endocrine disorders, metabolic disorders, kidney disease, or the like, the bone diseases including osteoporosis and osteoarthritis.
